# EUROPEAN PATENT APPLICATION

(11) **EP 4 063 370 A1**
(43) Date of publication of application: **28.09.2022**
(21) Application number: 21382232.3
(22) Date of filing: 23.03.2021
(51) Int. Cl.: C07D 491/14, C07D 498/14, A61P 35/00, A61K 31/5025

(54) **IMIDAZO[1,2-B]PYRIDAZINE BASED TRICYCLIC COMPOUNDS AS INHIBITORS OF HASPIN AND THERAPEUTIC USES THEREOF**

(71) Applicant: Fundación Centro Nacional de Investigaciones Oncológicas Carlos III, 28029 Madrid (ES)
(72) Inventor: PASTOR FERNÁNDEZ, Joaquín, 28029 Madrid (ES); MARTÍNEZ GONZÁLEZ, Sonia, 28029 Madrid (ES); BLANCO APARICIO, Carmen, 28029 Madrid (ES); GARCÍA GARCÍA, Ana Belén, 28029 Madrid (ES); RODRÍGUEZ ARÍSTEGUI, Sonsoles, 28029 Madrid (ES); GÓMEZ DE LA OLIVA, Cristina Ana, 28029 Madrid (ES); ALBARRÁN SANTIÑO, María Isabel, 28029 Madrid (ES); CEBRIÁ GÓMEZ, Antonio, 28029 Madrid (ES)
(74) Representative: Elzaburu S.L.P.

(57) **Abstract**

The present invention relates to a group of compounds with a tricyclic core based on imidazo[1,2-b]pyridazine of formula (I): 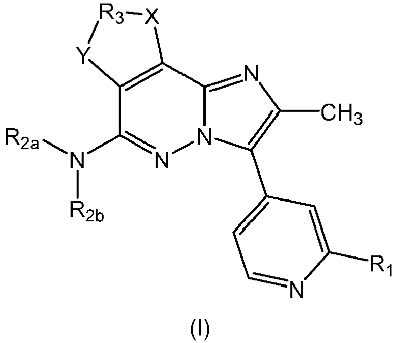 which are inhibitors of HASPIN, whose activity is required for the proliferation of certain tumoral cells, so the compounds of the invention are useful for the prevention and/or treatment of cancer.

## Description

### FIELD OF THE INVENTION

The present invention relates to a group of compounds with a tricyclic core based on imidazo[1,2-b]pyridazine, which are inhibitors of the protein kinase HASPIN. The compounds of the invention are useful for the treatment of cancer that depends on HASPIN. Therefore, the present invention belongs to the field of pharmacology or medicinal chemistry.

### BACKGROUND OF THE INVENTION

HASPIN (also known as germ cell-specific gene 2 protein/GSG2 or haploid germ cell-specific nuclear protein kinase) is a serine/threonine kinase. Its kinase domain shows a similar conformation to protein kinase ePK domain but diverges in crucial ways from typical ePK members. HASPIN lacks both the conserved ATP/Mg2+ binding motif Asp-Phe-Gly (DFG), which is replaced by Asp-Tyr-Thr (DYT), and the Ala-Pro-Glu (APE) motif usually found at the C terminus of the activation segment. HASPIN do not require phoshorylation of the activation loop to be active and the kinase domain of HASPIN alone is active *in vitro.* Accordingly, HASPIN is often classified as an atypical ePK family member.

HASPIN is overexpressed in some malignant tumors such as Burkitt's lymphoma, chronic lymphocytic leukemias (Dave et al., The New England Journal of Medicine. 2006, 354(23), 2431-2442), pancreatic cancer (PDAC) (Bastea et al., Sci Rep. 2019 Nov 12;9(1):16588), gallbladder carcinoma (GBC) (Zhu et al., Exp Cell Res. 2020 May 15;390 (2)), bladder cancer (Chen et al., Aging (Albany NY). 2020 May 21 ;12(10):8858-8879), prostate cancer (PCa) (Yu et al., Int J Oncol. 2020 Jul;57(1):139-150) and ovarian cancer (Huang et al., Oncogene.2020 May;39(21):4312-4322). In pancreatic cancer, patients with tumors with high HASPIN expression levels have shown a decrease in survival as compared to patients with tumors that express low levels of it. Moreover, the expression of HASPIN in GBC has been found up-regulated and positively correlated with the pathological grade of GBC. Also, HASPIN has been found up-regulated in bladder cancer tissues compared with the normal tissues and its high expression has been correlated with more advanced malignant grade and lower survival rate. Furthermore, HASPIN expression has been significantly associated with the development and progression of PCa. Finally, in ovarian cancer upregulation of HASPIN positively correlates with tumor grade and AJCC stage and negative correlated with patients' prognosis.

After HASPIN knockdown, the proliferation and clone-formation ability of GBC cells were inhibited as well as the growth of GBC *in vivo* (Zhu et al. Exp Cell Res. 2020 May 15;390 (2)). HASPIN knockdown in pancreatic cancer cells also inhibited cell proliferation, colony formation and migration, blocked cell cycle at G2 phase and induced cell apoptosis (Han, X. et al., Experimental Cell Research 2019, 385(1), 111605). In bladder cancer cells, the overexpression/knockdown of GSG2 promote/inhibit proliferation, colony formation and migration, while inhibiting/promoting cell apoptosis as well as knockdown of HASPIN suppress tumorigenicity of bladder cancer cells *in vivo.* KIF15 has been identified as the potential target of HASPIN in bladder cancer (Chen et al. 2020). Moreover, in prostate cancer cell lines, both *in vitro* and *in vivo,* HASPIN knockdown suppressed cell proliferation and colony formation promoting apoptosis (Yu et al. 2020). In addition, HASPIN has been identified in a whole kinome siRNA screen, together with Plk1, as one of the top hit kinases, whose depletion decreased both cell viability and estrogen receptor transcriptional activity in MCF7 breast cancer cells (Bhola et al., Cancer Research. 2015 Jan 15;75(2):405-414). Also, HASPIN has been described as one of the most upregulated proteins in FGF2-mediated resistance to EGFR inhibition. These cells are very sensitive to HASPIN inhibition and the combination of EGFR/HASPIN-i is much more effective that any treatment alone. Then, a combination of HASPIN-i and EGFR-i could be and option to prevent mitogen mediated resistance to EGFR-I (Koch et al., J. Proteome Res. 2016 Dec 2;15(12):4490-4504). Finally, HASPIN depletion has showed synthetic lethal interaction with VX-680 treatment (AURORA-i), specifically by inhibition of Aurora kinase B (Huang et al. Oncogene.2020 May;39(21):4312-4322).

The document WO2013/005041 refers to imidazo[1,2-b]pyridazine with a non-aromatic tricycle compounds which are lipid kinase inhibitors (such as PIM family), useful for the treatment of cancer or proliferative diseases. US2011/0312934 describes substituted imidazo[1,2-b]pyridazine compounds and their use in the treatment of prevention of diseases involving casein kinases 1-epsilon and/or 1-delta, such as cancer, among other diseases. The article J. Enzyme Inhib. and Med. Chem 2020, 35(1), 1840-1853, describes a series of imidazopyridazines based on the CHR-6494 which present inhibitory activity of Haspin and anti-proliferative properties against various human cancer cell lines.

Therefore, there is a clear need to obtain effective inhibitors of kinases whose activity is required for tumor cells proliferation, such as HASPIN, as it would be a good approach for the treatment of cancer.

### DESCRIPTION OF THE INVENTION

The present invention refers to a group of compounds with a tricyclic core based on imidazo[1,2-b]pyridazine, which are inhibitors of HASPIN. Regarding the role of HASPIN in certain tumoral cells as described above, these compounds are useful for the treatment and/or prevention of cancer.

Thus, in a first aspect, the invention refers to a compound of formula (I): 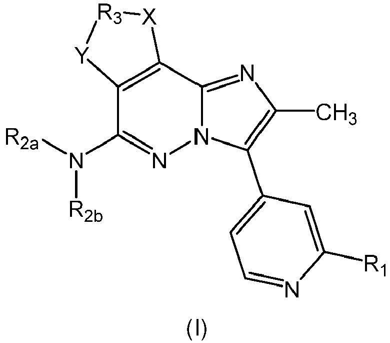 wherein:
R₁ is selected from the following groups: H, halo, CF₃,
R₂ₐ and R_{2b} are independently selected from the following groups:
   - H
   - alkyl C₁-C₆ substituted by OH or by heterocycle, said heterocycle being optionally substituted by halo;
   - cycloalkyl C₃-C₆ substituted by OH or by alkyl C₁-C₄;
or R₂ₐ and R_{2b} are linked and form a cycle together with the N atom to which they are attached, and R₂ₐ and R_{2b} are the same or different alkylene C₁-C₃ optionally substituted by an alkyl C₁-C₄, said alkyl being optionally substituted by OH or by NH₂;
X and Y are independently selected from O, CH₂, N(alkyl C₁-C₄) and R₃ is an alkylene C₁-C₂ optionally substituted by alkyl C₁-C₄, with the proviso that one of X and Y must be O and that the cycle formed by X, Y and R₃ is not aromatic;
or a pharmaceutically acceptable ester, amide, solvate or salt thereof.

Preferably, in the compound of formula (I) of the invention, R₁ is selected from Cl, F, CF₃.

Preferably, in the compound of formula (I) of the invention, R₂ₐ is H and R_{2b} is selected from alkyl C₁-C₄ substituted by OH or 4-piperidinyl optionally substituted by F.

Preferably, in the compound of formula (I) of the invention, R₂ₐ is H and R_{2b} is cycloalkyl C₆ substituted by OH and by alkyl C₁-C₂;
Preferably, in the compound of formula (I) of the invention, R₂ₐ and R_{2b} are linked and form a cycle together with the N atom to which they are attached, and R₂ₐ is alkylene C₃ and R_{2b} is alkylene C₂ optionally substituted by alkyl C₁-C₂ optionally substituted by NH₂.

Preferably, in the compound of formula (I) of the invention, X and Y are independently selected from O, CH₂, N-CH₃ and R₃ is an alkylene C₂ or alkylene C₁ optionally substituted by methyl.

Preferably, the compound of formula (I) of the invention is selected from the following list:
- [3-(2-Chloro-pyridin-4-yl)-2-methyl-7,8-dihydro-6H-9-oxa-1,3a,4-triaza-cyclopenta[a]naphthalen-5-yl]-(4-fluoro-piperidin-4-ylmethyl)-amine (1)
- C-{1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (2)
- C-{(S)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (3)
- C-{(R)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (4)
- 4-[3-(2-Chloro-pyridin-4-yl)-2-methyl-7,8-dihydro-6H-9-oxa-1,3a,4-triaza-cyclopenta[a]naphthalen-5-ylamino]-1-methyl-cyclohexanol (5)
- (4-Fluoro-piperidin-4-ylmethyl)-(2-methyl-3-pyridin-4-yl-7,8-dihydro-6H-9-oxa-1,3a,4-triaza-cyclopenta[a]naphthalen-5-yl)-amine (6)
- 4-[3-(2-Chloro-pyridin-4-yl)-2,7-dimethyl-6,7-dihydro-8-oxa-1,3a,4-triaza-as-indacen-5-ylamino]-1-methyl-cyclohexanol (7)
- [3-(2-Chloro-pyridin-4-yl)-2,6-dimethyl-7,8-dihydro-6H-9-oxa-1,3a,4,6-tetraaza-cyclopenta[a]naphthalen-5-yl]-piperidin-4-ylmethyl-amine (10)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-butan-1-ol (12)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (13)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (14)
- [2,6-Dimethyl-3-(2-trifluoromethyl-pyridin-4-yl)-7,8-dihydro-6H-9-oxa-1,3a,4,6-tetraaza-cyclopenta[a]naphthalen-5-yl]-piperidin-4-ylmethyl-amine (15)
- (2,6-Dimethyl-3-pyridin-4-yl-7,8-dihydro-6H-9-oxa-1,3a,4,6-tetraaza-cyclopenta[a]naphthalen-5-yl)-piperidin-4-ylmethyl-amine (17)
- 4-[3-(2-Fluoro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (18)
- [3-(2-Chloro-pyridin-4-yl)-2,9-dimethyl-8,9-dihydro-7H-6-oxa-1,3a,4,9-tetraaza-cyclopenta[a]naphthalen-5-yl]-(4-fluoro-piperidin-4-ylmethyl)-amine (25)
- 4-(2,9-Dimethyl-3-pyridin-4-yl-8,9-dihydro-7H-6-oxa-1,3a,4,9-tetraaza-cyclopenta[a]naphthalen-5-ylamino)-1-methyl-cyclohexanol (27)
- 4-[3-(2-Chloro-pyridin-4-yl)-2,9-dimethyl-8,9-dihydro-7H-6-oxa-1,3a,4,9-tetraaza-cyclopenta[a]naphthalen-5-ylamino]-1-methyl-cyclohexanol (28)
- 4-[2,9-Dimethyl-3-(2-trifluoromethyl-pyridin-4-yl)-8,9-dihydro-7H-6-oxa-1,3a,4,9-tetraaza-cyclopenta[a]naphthalen-5-ylamino]-1-methyl-cyclohexanol (29)
- [3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (33)
- [(R)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (34)
- [(S)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (35).

More preferably, the compound of formula (I) of the invention is selected from the following list:
- [3-(2-Chloro-pyridin-4-yl)-2-methyl-7,8-dihydro-6H-9-oxa-1,3a,4-triaza-cyclopenta[a]naphthalen-5-yl]-(4-fluoro-piperidin-4-ylmethyl)-amine (1)
- C-{1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (2)
- C-{(S)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (3)
- C-{(R)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (4)
- 4-[3-(2-Chloro-pyridin-4-yl)-2-methyl-7,8-dihydro-6H-9-oxa-1,3a,4-triaza-cyclopenta[a]naphthalen-5-ylamino]-1-methyl-cyclohexanol (5)
- (4-Fluoro-piperidin-4-ylmethyl)-(2-methyl-3-pyridin-4-yl-7,8-dihydro-6H-9-oxa-1,3a,4-triaza-cyclopenta[a]naphthalen-5-yl)-amine (6)
- 4-[3-(2-Chloro-pyridin-4-yl)-2,7-dimethyl-6,7-dihydro-8-oxa-1,3a,4-triaza-as-indacen-5-ylamino]-1-methyl-cyclohexanol (7)
- [3-(2-Chloro-pyridin-4-yl)-2,6-dimethyl-7,8-dihydro-6H-9-oxa-1,3a,4,6-tetraaza-cyclopenta[a]naphthalen-5-yl]-piperidin-4-ylmethyl-amine (10)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-butan-1-ol (12)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (13)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (14)
- [3-(2-Chloro-pyridin-4-yl)-2,9-dimethyl-8,9-dihydro-7H-6-oxa-1,3a,4,9-tetraaza-cyclopenta[a]naphthalen-5-yl]-(4-fluoro-piperidin-4-ylmethyl)-amine (25)
- 4-(2,9-Dimethyl-3-pyridin-4-yl-8,9-dihydro-7H-6-oxa-1,3a,4,9-tetraaza-cyclopenta[a]naphthalen-5-ylamino)-1-methyl-cyclohexanol (27)
- 4-[3-(2-Chloro-pyridin-4-yl)-2,9-dimethyl-8,9-dihydro-7H-6-oxa-1,3a,4,9-tetraaza-cyclopenta[a]naphthalen-5-ylamino]-1-methyl-cyclohexanol (28)
- [3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (33)
- [(R)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (34)
- [(S)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (35).

Another aspect of the invention refers to compound of formula (I) as previously described for use as a medicament.

Another aspect of the invention refers to compound of formula (I) as previously described for use in the prevention or treatment of cancer.

More preferably, the cancer is selected from Burkitt's lymphoma, chronic lymphocytic leukemias, pancreatic cancer, gallbladder carcinoma, bladder cancer, prostate cancer, melanoma, breast cancer, ovarian cancer.

Another aspect of the invention refers to a composition comprising a compound of formula (I) as previously described and a pharmaceutically acceptable excipient, diluent or carrier.

In the present invention, the term "alkyl" refers to linear or branched hydrocarbonated chain radicals, with between 1 and 6 carbon atoms, preferably between 1 and 4, which bind to the rest of the molecule by means of a single bond, for example, propyl, ethyl, methyl, isopropyl, butyl, pentyl, etc. These alkyl radicals may be optionally substituted in one or more positions by one or more groups, such as cycloalkyl, hydroxyl, amines, amides, oxo, cyano, halogens, aryl, etc.

In the present invention, the term "alkylene" refers to a bivalent saturated aliphatic radical regarded as derived from an alkane by removal of two hydrogen atoms from different carbon atoms, with between 1 and 4 carbon atoms, preferably between 1 and 2, which binds to the rest of the molecule by means of two single bonds, for example, ethylene, propylene, isopropylene, etc. These alkyl radicals may be optionally substituted in one or more positions by one or more groups, such as cycloalkyl, hydroxyl, amines, amides, oxo, cyano, halogens, aryl, etc.

In the present invention, the term "cycloalkyl" refers to non-aromatic monocyclic or polycyclic ring comprising carbon and hydrogen atoms, preferably with between 3 and 6 carbon atoms, and more preferably 6, totally or partially saturated, and formed by only carbon and hydrogen atoms. They may be optionally substituted by one or more groups, such as alkyl, halogens, hydroxyl, amines, amides, cyano, etc. Examples of alkyl groups include, but are not limited to, cyclopropyl, cyclobutyl, cyclopentyl or cyclohexyl.

In the present invention, the term "heterocycle" refers to non-aromatic monocyclic or polycyclic ring comprising carbon and hydrogen atoms and at least one heteroatom (nitrogen, oxygen or sulfur). Preferably is a 4 to 8-member ring with one or more heteroatoms, more preferably is a 5 or 6-member ring with one or more heteroatoms. They may be optionally substituted by one or more groups, such as alkyl, halogens, hydroxyl, amines, amides, cyano, etc. Examples of heterocycle groups include, but are not limited to, piperazinyl, pyrrolidinyl, dioxanyl, morpholinyl, tetrahydrofuranyl, etc.

"Halo" or "halogen" refers to fluorine, chlorine, bromine or iodine.

The compounds of the present invention represented by the formula (I), and more specifically, the specific compounds belonging to this previously described general formula may include isomers, depending on the presence of multiple bonds (for example, Z, E), including optic isomers or enantiomers, depending on the presence of chiral centres. The individual isomers, enantiomers or diastereoisomers and the mixtures thereof fall within the scope of the present invention. The individual enantiomers or diastereoisomers, as well as their mixtures, can be separated by conventional techniques.

The compounds of the invention may be in crystalline form as free compounds or in solvate form, intending both forms to be within the scope of the present invention. In this sense, the term "solvate", as used herein, includes both pharmaceutically acceptable solvates, in other words, solvates of the compound of formula (I) which can be used to produce a medicament, as well as pharmaceutically unacceptable solvates, which can be useful to produce pharmaceutically acceptable solvates or salts. The nature of the pharmaceutically acceptable solvate is not critical on condition that it is pharmaceutically acceptable. In a particular embodiment, the solvate is a hydrate. The solvates can be obtained by conventional solvation methods known to technicians in the art.

For therapeutic application, the compounds of formula (I), their salts or solvates, will come preferably in a pharmaceutically acceptable or substantially pure form, in other words, having a pharmaceutically acceptable level of purity excluding standard drugs such as diluents and carriers, and not including material considered toxic at standard dose levels. The purity levels for the active principle are preferably higher than 50%, more preferably higher than 70%, more preferably higher than 90%. In a preferred embodiment, they are higher than 95% of the compound of formula (I), or the salts or solvates thereof.

Another aspect of the invention refers to a composition comprising a compound of formula (I) as described above and a pharmaceutically acceptable excipient, diluent or carrier. Preferably, said composition further comprises an antitumoral compound, which may be any chemotherapeutic agent authorized to be used in humans or in the process of clinical trials and/or pending of said authorization. Examples of said chemotherapeutic agents may be found, for example, in patent EP3341376.

The term "excipients, diluent or carriers" relates to molecular entities or substances through which the active ingredient is administered. Such pharmaceutical excipients, diluents or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes. Suitable pharmaceutical excipients and carriers are known in the art by a skilled person.

The compounds of formula (I) for therapeutic use are prepared in solid form or aqueous suspension, in a pharmaceutically acceptable diluent. These preparations may be administered via any appropriate route of administration, wherefore said preparation will be formulated in the suitable pharmaceutical form for the selected route of administration. In a particular embodiment, the compound of formula (I) provided by this invention is administered orally, topically, rectally or parenterally (including subcutaneously, intraperitoneally, intradermally, intramuscularly, intravenously, etc.). A review of the different pharmaceutical forms for administering medicaments and the excipients required to obtain them can be found in the standard Pharmacopoeias of Europe and the US.

The compounds described in the present invention, their pharmaceutically acceptable salts, and solvates, as well as the pharmaceutical compositions containing them can be used in conjunction with other additional drugs in order to provide a combination therapy. Said additional drugs may form part of the same pharmaceutical composition or, alternatively, may be provided in the form of a separate composition for administration simultaneously or not with the administration of the pharmaceutical composition comprising a compound of formula (I), or pharmaceutically acceptable solvate or salt thereof.

Another additional aspect of the present invention refers to a method for treating cancer, comprising the administration of a therapeutically effective amount of a compound of formula (I) as described above.

In the sense used in this description, the expression "therapeutically effective amount" refers to the quantity of agent or compound capable of having an effect on the levels of proliferation in primary cultured cells, calculated to produce the required effect in vivo and, in general, will be determined, among other aspects, by the inherent properties of the compounds, including the age, state of the patient, severity of the alteration or disorder, and route and frequency of administration. In general, the therapeutically effective amount of the compound of formula (I) to be administered will depend, among other factors, on the individual who is to be treated, the severity of the disease suffered by the individual, the selected form of administration, etc. For this reason, the doses mentioned in this invention must be considered solely as guides for the skilled person, who must adjust the doses according to the aforementioned variables. However, a compound of formula (I) may be administered one or more times a day, for example 1, 2, 3 or 4 times a day, in a typical total daily quantity comprised between 0.1 and 1,000 mg/kg body weight/day, preferably 10 mg/kg body mass/day.

The compound of the invention is compatible for use in protocols wherein the compounds of formula (I) or their mixtures are used alone or in combination with other treatments or medical procedures, such as radiotherapy or immunotherapy.

All individual features (e.g. preferred features) mentioned herein may be taken in isolation or in combination with any other feature (including a preferred feature) mentioned herein (hence, preferred features may be taken in conjunction with other preferred features, or independently of them).

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practice of the invention. The following examples and figures are provided for illustrative purposes and are not intended to limit the scope of this invention.

### EXAMPLES

### A. General methods for the synthesis of the compounds of the invention

### Example 1. Compound Nr. 1: [3-(2-Chloro-pyridin-4-yl)-2-methyl-7,8-dihydro-6H-9-oxa-1,3a,4-triaza-cyclopenta[a]naphthalen-5-yl]-(4-fluoro-piperidin-4-ylmethyl)-amine

Intermediate **VII** (40 mg, 0.072 mmol) in DCM (2 mL) was treated with TFA (80 µL, 1.08 mmol) and the mixture was stirred at rt for 2 h 30 min. The mixture was concentrated under vacuum and the residue was purified by column chromatography on SCX-2 cartridge using a solvent gradient from 0% to 10% of NH₃ (7N in MeOH) in MeOH to afford 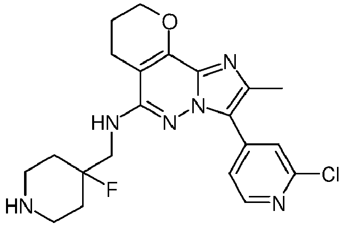 Compound Nr. **1** (white solid, 25 mg, 81%). LCMS (ESI): Rt = 4.05min, m/z = 431.10 = [M+H]⁺. ¹H NMR (300 MHz, DMSO-*d6*) δ ppm 8.45 (d, *J* = 5.3 Hz, 1H), 8.17 (d, *J* = 0.9 Hz, 1H), 7.75 (dd, *J* = 5.4, 1.5 Hz, 1H), 6.39 (t, *J* = 6.2 Hz, 1H), 4.39 - 4.27 (m, 2H), 3.56 (dd, *J* = 20.7, 6.1 Hz, 2H), 3.34 (s, 3H), 2.70 (m, 4H), 2.50 (2H under dmso signal), 2.81 - 2.61 (m, 4H), 2.04 (dd, *J* = 12.7, 8.0 Hz, 2H), 1.71 (dd, *J* = 23.9, 11.8 Hz, 3H), 1.62 - 1.46 (m, 1H).

### Example 2. Compound Nr. 2: C-{1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine

Intermediate **X** (25 mg, 0.049 mmol) in DCM (1mL) was treated with TFA (75 µL, 0.98 mmol). The mixture was stirred at rt overnight. The mixture was concentrated under vacuum and the residue was purified by column chromatography on SCX-2 cartridge using a solvent gradient from 0% to 10% of NH₃ (7N in MeOH) in MeOH and then by HPLC to yield Compound Nr. **2** (white solid, 6mg, 30%). LCMS (ESI): Rt = 5.12 min, m/z = 413.20 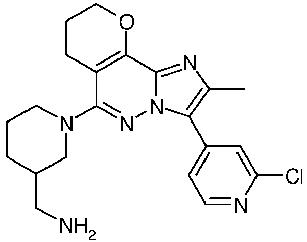 = [M+H]⁺. ¹H NMR (300 MHz, DMSO-d6) δ ppm 8.48 (d, *J*= 5.3 Hz, 1H), 8.06 (d, *J*= 1.0 Hz, 1H), 7.83 (dd, *J*= 5.4, 1.4 Hz, 1H), 4.55 - 4.32 (m, 2H), 3.54 (d, *J*= 11.6 Hz, 1H), 3.38 (d, *J*= 12.5 Hz, 1H), 2.78 (dd, *J* = 20.6, 8.6 Hz, 1H), 2.76 - 2.60 (m, 3H), 2.53 (s, 3H), 2.50 (2H under dmso signal), 2.06 - 1.71 (m, 5H), 1.61 (d, *J* = 11.5 Hz, 1H), 1.18 (m, 1H).

### Example 3. Compound Nr. 3: C-{(S)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine

Intermediate **XIII** (28 mg, 0.055 mmol) in DCM (2 mL) was treated with TFA (63 µL, 0.825 mmol) and the mixture was stirred at rt for 2 h 30 min. The mixture was concentrated under vacuum and the residue was purified by column chromatography on SCX-2 cartridge using a solvent gradient from 0% to 10% of NH₃ (7N in MeOH) in MeOH and then by HPLC to render Compound Nr. **3** as formic acid salt (white solid, 6 mg, 24 %). LCMS (ESI): Rt = 2.47 min, m/z = 413.20 = [M+H]⁺. *ee* = 100% determined by HPLC on Chiral IC; Hept/EtOH/ EDA: 70/30/0.1; 89.7min. ¹H NMR (300 MHz, DMSO-*d6*) δ ppm 8.48 (d, J = 5.3 Hz, 1H), 8.33 (s, 1H), 8.05 (d, *J* = 1.0 Hz, 1H), 7.83 (dd, *J* = 5.4, 1.4 Hz, 1H), 4.42 (m, 2H), 3.53 (d, *J* = 12.0 Hz, 1H), 3.37 (d, *J* = 12.3 Hz, 1H), 2.91 - 2.55 (m, 1H), 2.76 - 2.60 (m, 4H), 2.53 (s, 3H), 2.50 (2H under dmso signal), 2.06 - 1.71 (m, 5H), 1.62 (d, *J* = 9.9 Hz, 1H), 1.18(m, 1H).

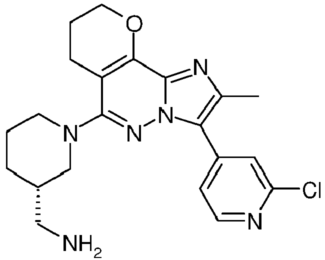

### Example 4. Compound Nr. 4: C-{(R)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine

Intermediate **XVI** (46 mg, 0.090 mmol) in DCM (3 mL) was treated with TFA (0.15 mL, 2.25 mmol). The mixture was stirred at rt for 4 h and then it was concentrated under vacuum. The residue was purified first by column chromatography on SCX-2 cartridge using a solvent gradient from 0% to 5% of NH₃ (7N in MeOH) in MeOH and then by HPLC to render Compound Nr. **4** as formic acid salt (white solid, 23 mg, 56%). LCMS (ESI): Rt = 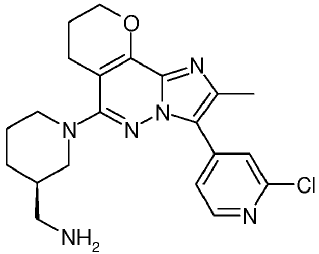 2.37 min, m/z = 413.20 = [M+H]⁺. *ee* = 100% determined by HPLC on Chiral IC; Hept/EtOH/ EDA: 70/30/0.1; 76.6min. ¹H NMR (300 MHz, DMSO-d6) δ ppm 8.48 (d, *J* = 5.1 Hz, 1H), 8.37 (s, 1H), 8.07 (d, *J* = 1.0 Hz, 1H), 7.83 (dd, *J*= 5.4, 1.4 Hz, 1H), 4.62 - 4.27 (m, 2H), 3.53 - 3.37 (2H under water signal), 2.91 - 2.55 (m, 2H), 2.74 - 2.63 (m, 2H), 2.54 (s, 3H), 2.50 (2H under dmso signal), 1.89- 1.72 (m, 2H), 1.62 (d, *J* = 9.9 Hz, 1H), 1.18 (m, 1H).

### Example 5. Compound Nr. 5: 4-[3-(2-Chloro-pyridin-4-yl)-2-methyl-7,8-dihydro-6H-9-oxa-1,3a,4-triaza-cyclopenta[a]naphthalen-5-ylamino]-1-methyl-cyclohexanol

Intermediate **XVIII** (39 mg, 0.15 mmol), 2-chloropyridine-4-boronic acid (23 mg, 0.225 mmol), PdCl₂dppf (8 mg, 0.015 mmol) and Cs₂CO₃ (64 mg, 0.30 mmol) were mixed in dioxane and the mixture in pressure tube was heated at 120 ºC for 2 h 30 min. The mixture was filtered through Celite pad and concentrated under vacuum. The residue was purified by column chromatography on silica gel using a solvent gradient from 0% to 5% of MeOH in EtOAc and then 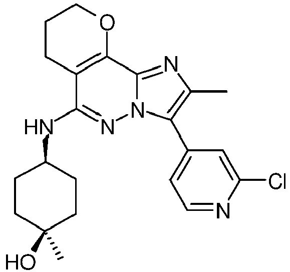 by HPLC to render Compound Nr. **5** (white solid, 3 mg, 7%). LCMS (ESI): Rt = 3.55 min, m/z = 428.20 = [M+H]⁺. ¹H NMR (300 MHz, DMSO-*d6*) δ ppm 8.43 (d, *J* = 5.4 Hz, 1H), 8.21 (d, *J* = 1.0 Hz, 1H), 7.73 (dd, *J* = 5.4, 1.5 Hz, 1H), 5.93 (d, *J* = 7.4 Hz, 1H), 4.35 - 4.26 (m, 2H), 3.55 (br s, 1H partially under water signal), 2.50 (3H under dmso signal), 2.43 (dd, *J* = 8.5, 4.8 Hz, 2H), 2.03 (dt, *J*= 14.7, 5.3 Hz, 2H), 1.74 (m, 4H), 1.61 (m, 2H), 1.43 (m, 2H), 1.13 (s, 3H).

### Example 6. Compound Nr. 6: (4-Fluoro-piperidin-4-ylmethyl)-(2-methyl-3-pyridin-4-yl-7,8-dihydro-6H-9-oxa-1,3a,4-triaza-cyclopenta[a]naphthalen-5-yl)-amine

Intermediate **XIX** (45 mg, 0.091 mmol) in DCM (2 mL) was treated with TFA (0.1 mL, 1.365 mmol) and the mixture was stirred at rt for 2 h. The mixture was concentrated under vacuum. The residue was purified by column chromatography on SCX-2 cartridge using a solvent gradient from 0% to 5% of NH₃ (7N in MeOH) in MeOH to render Compound Nr. **6** (white solid, 28 mg, 78%). LCMS (ESI): Rt = 2.56 min, m/z = 397.20 = [M+H]⁺. ¹H 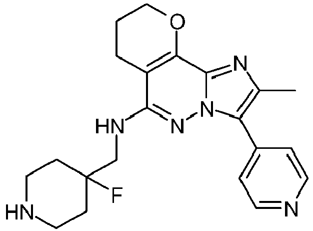 NMR (300 MHz, DMSO-d6) δ ppm 8.63 (dd, *J* = 4.6, 1.6 Hz, 2H), 7.84 (dd, *J* = 4.6, 1.6 Hz, 12H), 6.26 (t, *J* = 6.2 Hz, 1H), 4.40 - 4.23 (m, 2H), 3.55 (dd, *J* = 20.5, 6.2 Hz, 2H), 2.70 (m, 4H), 2.50 (m, 2H under dmso signal), 2.46 (s, 3H), 2.05 (m, 2H), 1.73 - 1.41 (m, 4H).

### Example 7. Compound Nr. 7: 4-[3-(2-Chloro-pyridin-4-yl)-2,7-dimethyl-6,7-dihydro-8-oxa-1,3a,4-triaza-as-indacen-5-ylamino]-1-methyl-cyclohexanol

Intermediate **XXIII** (50 mg, 0.126 mmol), 2-chloropyridine-4-boronic acid (30 mg, 0.189 mmol), Cs₂CO₃ (68 mg, 0.252 mmol) and PdCl₂dppf (10 mg, 0.013 mmol) were mixed in dioxane (1.5 mL) and water (0.15 mL) and heated in pressure tube at 120 ºC for 2 h 30 min. The mixture was taken in ethyl acetate and water was added. The layers were separated and the aqueous phase was extracted with ethyl acetate. The combined organic extract was dried and concentrated. The residue was purified by column chromatography 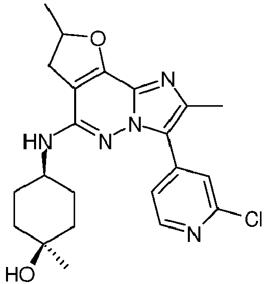 on silica gel using a solvent gradient from 0% to 5% of MeOH in EtOAc and then it was re-purified using gradient from 0% to 5% of MeOH in DCM. Additional purification by HPLC afforded Compound Nr. **7** (white solid, 8 mg, 14%). LCMS (ESI): Rt = 4.19 min, m/z = 482.20 = [M+H]⁺. ¹H NMR (300 MHz, DMSO-d6) δ ppm 8.45 (d, *J* = 5.4 Hz, 1H), 8.21 (d, *J* = 0.9 Hz, 1H), 7.74 (dd, *J* = 5.4, 1.5 Hz, 1H), 6.37 (d, *J* = 7.4 Hz, 1H), 5.36 - 5.14 (m, 1H), 4.07 (s, 1H), 3.56 (m, 1H), 3.29 - 3.23 (m, 2H), 2.80 - 2.65 (m, 2H), 2.50 (3H, under dmso signal), 1.69 (m, 4H), 1.61 (m, 2H), 1.47 (d, *J*= 6.3 Hz, 3H), 1.13 (s, 3H).

### Example 8. Compound Nr. 10: [3-(2-Chloro-pyridin-4-yl)-2,6-dimethyl-7,8-dihydro-6H-9-oxa-1,3a,4,6-tetraaza-cyclopenta[a]naphthalen-5-yl]-piperidin-4-ylmethyl-amine

To a solution of Intermediate **XXX** (26 mg, 0.05 mmo) in dioxane (1.5 mL) was added 4 M HCl in dioxane (0.3 mL). The resulting mixture was stirred for 18 h. Solvents were removed under vacuum. The residue was purified by column chromatography on silica using first a solvent gradient from 0% to 5% of MeOH in DCM and after from 5% to 10% of NH₃ (7N in MeOH) in DCM. The required product was recovered 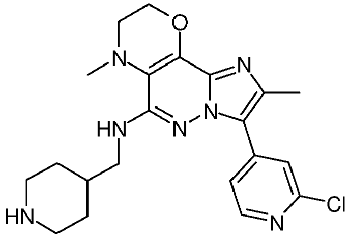 from the column and it was triturated with diethyl ether twice, to render Compound Nr. **10** (white solid, 4 mg, 19%). LCMS (ESI): Rt = 0.35 min, m/z = 428.30 [M+H]⁺. ¹H NMR (300 MHz, DMSO-*d6*) δ ppm 8.44 (d, *J* = 5.1 Hz, 1H), 8.13 (s, 1H), 7.76 (d, *J* = 4.8 Hz, 1H), 6.70 - 6.61 (m, 1H), 4.38 (d, *J* = 3.7 Hz, 2H), 3.28 - 3.08 (m, 4H), 2.91 - 2.78 (m, 1H), 2.65 (s, 3H), 2.45 (3H, under dmso signal), 2.04 - 1.94 (m, 2H), 1.92 - 1.80 (m, 2H), 1.50 - 1.28 (m, 4H).

### Example 9. Compound Nr. 12: 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-butan-1-ol

A mixture of Intermediate **XXXII** (62 mg, 0.15 mmol), 2-chloropyridine-4-boronic acid (30 mg, 0.20 mmol), Cs₂CO₃ (160 mg, 0.47 mmol), Pd(dppf)Cl₂ (15 mg) in dioxane (1.5 mL) and water (0.2 mL) was heated in a sealed tube at 120 ºC for 5 h. The dark mixture was cooled down, filtered through a Celite pad and concentrated under vacuum. The residue was 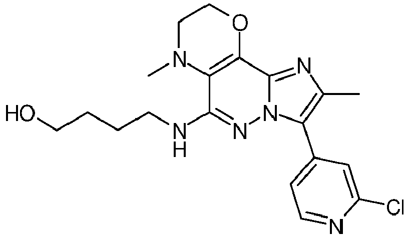 purified by column chromatography on silica gel using a solvent gradient from 25% to 100% of EtOAc in hexanes and 5% of MeOH. The required product obtained was triturated with diethyl ether to render Compound Nr. **12** (white solid, 18 mg, 30%). LCMS (ESI): Rt = 3.04 min, m/z = 403.10 [M+H]⁺. ¹H NMR (300 MHz, DMSO-d6) δ ppm 8.42 (d, *J* = 5.3 Hz, 1H), 8.12 (d, *J* = 1.0 Hz, 1H), 7.78 (dd, *J* = 5.4, 1.5 Hz, 1H), 6.40 (t, *J* = 5.6 Hz, 1H), 4.42 (t, *J* = 5.1 Hz, 1H), 4.39 - 4.32 (m, 2H), 3.44 - 3.36 (m, 2H), 3.32 - 3.18 (m, 2H), 3.18 - 3.08 (m, 2H), 2.64 (s, 3H), 2.48 (3H,under dmso signal), 1.77 - 1.57 (m, 2H), 1.58 - 1.42 (m, 2H).

### Example 10. Compound Nr. 13: 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol

A mixture of Intermediate **XXXIV** (40 mg, 0.08 mmol), 2-chloropyridine-4-boronic acid (20 mg, 0.1 mmol), Pd(dppf)Cl₂ (8 mg), Cs₂CO₃ (85 mg, 0.3 mmol) in dioxane (1 mL) and water (0.2 mL) was heated in a sealed tube at 100 ºC for 3 h. The dark mixture was cooled down, filtered through a Celite pad rinsing with DCM and concentrated under vacuum. The crude was purified by column chromatography on silica gel using a solvent gradient from 0% to 5% of MeOH in EtOAc. The product obtained was triturated with diethyl 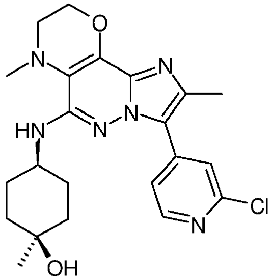 ether to render Compound Nr. **13** (cream solid, 9 mg, 23%). LCMS (ESI): Rt = 3.38 min, m/z = 443.20 [M+H]⁺. ¹H NMR (300 MHz, DMSO-d6) δ ppm 8.42 (d, *J* = 5.4 Hz, 1H), 8.18 (d, *J* = 1.0 Hz, 1H), 7.71 (dd, *J* = 5.4, 1.5 Hz, 1H), 5.72 (d, *J* = 7.0 Hz, 1H), 4.39 - 4.29 (m, 2H), 4.07 (s, 1H), 3.71 - 3.49 (m, 1H), 3.19 - 3.11 (m, 2H), 2.64 (s, 3H), 2.48 (3H,under dmso signal), 1.85 - 1.67 (m, 4H), 1.68 - 1.53 (m, 2H), 1.50 - 1.33 (m, 2H), 1.13 (s, 4H).

### Example 11. Compound Nr. 14: 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol

A mixture of Intermediate **XXXVI** (20 mg, 0.04 mmol), 2-chloropyridine-4-boronic acid (10 mg, 0.05 mmol), Cs₂CO₃ (30 mg, 0.09 mmol), Pd(dppf)Cl₂ (5 mg) in dioxane (0.5 mL) and water (0.05 mL) was heated in a sealed tube for 3 h. The dark mixture was cooled down and filtered through a Celite pad rinsing with DCM. The filtrate was concentrated and the crude was purified by column chromatography on silica gel using a solvent gradient from 25% to 100% of EtOAc in cHex and 10% of MeOH. The product obtained 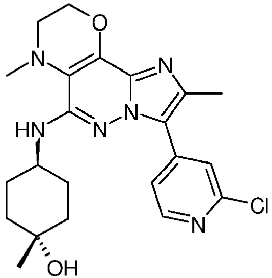 was further purified by preparative. HPLC to render Compound Nr. **14** as formic acid salt (white solid, 5 mg, 25%). LCMS (ESI): Rt = 3.44 min, m/z = 443.20 [M+H]⁺. ¹H NMR (300 MHz, DMSO-*d6*) δ ppm 8.42 (d, *J* = 5.4 Hz, 1H), 8.18 (d, *J* = 1.0 Hz, 1H), 7.71 (dd, *J* = 5.4, 1.5 Hz, 1H), 5.72 (d, *J* = 7.0 Hz, 1H), 4.39 - 4.29 (m, 2H), 4.07 (s, 1H), 3.71 - 3.49 (m, 1H), 3.19 - 3.11 (m, 2H), 2.64 (s, 3H), 2.48 (3H,under dmso signal), 1.85 - 1.67 (m, 4H), 1.68 - 1.53 (m, 2H), 1.50 - 1.33 (m, 2H), 1.13 (s, 3H).

### Example 12. Compound Nr. 15: [2,6-Dimethyl-3-(2-trifluoromethyl-pyridin-4-yl)-7,8-dihydro-6H-9-oxa-1,3a,4,6-tetraaza-cyclopenta[a]naphthalen-5-yl]-piperidin-4-ylmethyl-amine

A mixture of Intermediate **XXXVII** (15 mg, 0.02 mmol) in dioxane (1 mL) and 4 M HCl in dioxane (0.1 mL) was stirred for 24 h. Solvents were removed under vacuum, and the residue was purified by column chromatography on silica gel using a solvent gradient first from 0% to 5% of MeOH in DCM and then gradient from 5% to 10% of NH₃ (7N in MeOH) in DCM to give the required Product **15** (white solid, 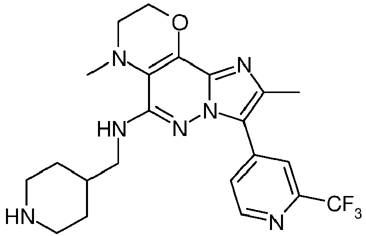 7 mg, 52%). LCMS (ESI): Rt = 0.37 min, m/z = 462.20 [M+H]⁺. ¹H NMR (300 MHz, DMSO-d6) δ ppm d 8.79 (t, *J* = 5.0 Hz, 1H), 8.55 (s, 1H), 8.01 (d, *J* = 5.3 Hz, 1H), 6.59 (t, *J* = 5.6 Hz, 1H), 4.42 - 4.34 (m, 2H), 3.26 - 3.13 (m, 5H), 2.92 - 2.74 (m, 2H), 2.66 (s, 3H), 2.44 (3H,under dmso signal), 2.10 - 1.93 (m, 2H), 1.86 - 1.73 (m, 2H), 1.40 - 1.28 (m, 2H).

### Example 13. Compound Nr. 17: (2,6-Dimethyl-3-pyridin-4-yl-7,8-dihydro-6H-9-oxa-1,3a,4,6-tetraaza-cyclopenta[a]naphthalen-5-yl)-piperidin-4-ylmethyl-amine

A mixture of Intermediate **XXXVIII** (45 mg, 0.09 mmol) in dioxane (1 mL) and 4 M HCI in dioxane (0.150 mL) was stirred for 24 h. Solvents were removed in vacuum, and the residue was purified by column chromatography on silica gel using a solvent gradient first from 0% to 5% of MeOH in DCM and then a gradient from 5% to 10% of NH₃ (7N in MeOH) in DCM to give the required Compound Nr. **17** (white solid, 5 mg, 15%). LCMS (ESI): Rt = 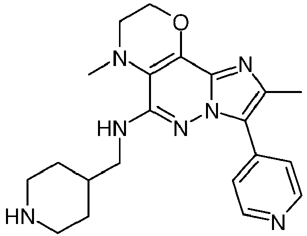 0.42 min, m/z = 394.20 [M+H]⁺. ¹H NMR (300 MHz, DMSO-*d6*) δ ppm 8.67 (d, *J* = 6.1 Hz, 2H), 7.92 (d, *J* = 3.7 Hz, 2H), 4.38 (d, *J* = 4.0 Hz, 2H), 3.24 - 3.12 (m, 5H), 2.92 - 2.76 (m, 1H), 2.66 (s, 2H), 2.47 (3H, under dmso signal), 2.12 - 1.93 (m, 2H), 1.90 - 1.74 (m, 2H), 1.40 - 1.28 (m, 2H).

### Example 14. Compound Nr. 18: 4-[3-(2-Fluoro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol

A mixture of Intermediate **XXXIV** (40 mg, 0.08 mmol), 2-fluoropyridine-4-boronic acid pinacol ester (25 mg, 0.1 mmol), Pd(dppf)Cl₂ (15 mg), Cs₂CO₃ (85 mg, 0.3 mmol) in dioxane (1 mL) and water (0.2 mL) was heated at 110 ºC for 2 h. The dark mixture was cooled down, filtered through a Celite pad rinsing with DCM and concentrated under vacuum. The crude obtained was purified by column chromatography on silica gel using a solvent gradient from 25% to 100% of EtOAc in cHex and with 5% of MeOH to give the 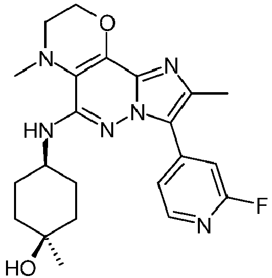 product that was purified again by preparative HPLC yielding the Compound Nr. **18** as formic acid salt (white solid, 11 mg, 29%). LCMS (ESI): Rt = 3.30 min, m/z = 427.30 [M+H]⁺. ¹H NMR (300 MHz, DMSO-*d6*) δ ppm 8.26 (d, *J* = 5.4 Hz, 1H), 7.77 (s, 1H), 7.73 (dd, *J* = 5.4, 1.9 Hz, 1H), 5.79 - 5.69 (m, 1H), 4.43 - 4.31 (m, 2H), 4.07 (s, 1H), 3.64 - 3.49 (m, 1H), 3.19 - 3.11 (m, 2H), 2.65 (s, 3H), 2.46 (s, 3H under dmso signal), 1.87 - 1.71 (m, 4H), 1.70 - 1.53 (m, 2H), 1.47 - 1.33 (m, 2H), 1.14 (s, 3H).

### Example 15. Compound Nr. 25: [3-(2-Chloro-pyridin-4-yl)-2,9-dimethyl-8,9-dihydro-7H-6-oxa-1,3a,4,9-tetraaza-cyclopenta[a]naphthalen-5-yl]-(4-fluoro-piperidin-4-ylmethyl)-amine

Intermediate **XLI** (46 mg; 0.084 mmol) in DCM (2 mL) was treated with TFA (0.25 mL, 2.106 mmol) and stirred for 90 min. The mixture was diluted with DCM and washed with aqueous NaHCO₃ solution. The organic phase was dried over Na₂SO₄, filtered and concentrated. The residue was purified by column chromatography on silica gel using a solvent gradient from 0% to 10% of MeOH in DCM and then 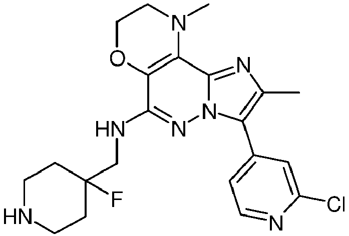 by HPLC to yield Compound Nr. **25** as formic acid salt (yellow solid, 5 mg, 13%). LCMS (ESI): Rt = 2.10 min, m/z = 446.20 = [M+H]⁺. ¹H NMR (300 MHz, DMSO-d6) δ ppm 8.44 (d, *J* = 5.3 Hz, 1H), 8.34 (s, 1H), 8.11 (s, 1H), 7.74 (d, *J* = 5.1 Hz, 1H), 6.30 (t, *J* = 5.8 Hz, 1H), 4.30 (s, 2H), 3.54 (s, 3H), 3.60 - 3.46 (m, 2H), 3.41 (m, 2H), 3.07 - 2.90 (m, 2H), 2.83 (m, 2H), 2.50 (s, 3H under dmso signal), 1.82 (m, 3H), 1.72 (m, 1H).

### Example 16. Compound Nr. 27: 4-(2,9-Dimethyl-3-pyridin-4-yl-8,9-dihydro-7H-6-oxa-1,3a,4,9-tetraaza-cyclopenta[a]naphthalen-5-ylamino)-1-methyl-cyclohexanol

A mixture of Intermediate **XLV** (64 mg, 0.15 mmol), pyridine-4-boronic acid pinacol ester (48 mg, 0.225 mmol), Pd(dppf)Cl₂ (13 mg, 0.015 mmol) and Cs₂CO₃ (102 mg, 0.30 mmol) in 1,4-dioxane (1.5 mL) and H₂O (0.2 mL) was heated at 100 ºC in a sealed tube for 18 h. The mixture was partitioned between H₂O and DCM. The aqueous layer was extracted with DCM (x3). The combined organic extracts were dried (Na₂SO₄), filtered and concentrated under vacuum. The residue was purified by column chromatography on silica gel using a solvent 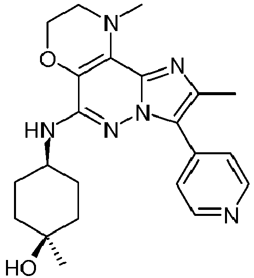 gradient from 0% to 5% of MeOH in DCM and then it was re-purified using gradient from 20% to 70% of EtOAc/MeOH 9:1 in cHex to render Compound Nr. **27** (white solid, 14 mg; 22%). LCMS (ESI): Rt = 6.36 and 6.66 min, m/z = 409.20 = [M+H]⁺. ¹H NMR (300 MHz, DMSO-*d6*) δ ppm 8.65 (dd, *J* = 4.6, 1.6 Hz, 2H), 7.89 (dd, *J* = 4.7, 1.6 Hz, 2H), 5.67 (d, *J* = 7.2 Hz, 1H), 4.35 - 4.32 (m, 2H), 4.11 (s, 1H), 3.58 (s, 3H), 3.30 (3H under water signal), 2.64 - 2.62 (m, 1H), 2.53 (s, 3H), 1.84 - 1.63 (m, 6H), 1.47 - 1.41 (m, 2H), 1.20 (s, 3H).

### Example 17. Compound Nr. 28: 4-[3-(2-Chloro-pyridin-4-yl)-2,9-dimethyl-8,9-dihydro-7H-6-oxa-1,3a,4,9-tetraaza-cyclopenta[a]naphthalen-5-ylamino]-1-methyl-cyclohexanol

A mixture of Intermediate **XLV** (47 mg, 1 eq, 0.115 mmol), 2-chloropyridine-4-boronic acid pinacol ester (27 mg, 0.172 mmol), Pd(dppf)Cl₂ (9 mg, 0.012 mmol) and Cs₂CO₃ (75 mg, 0.23 mmol) in 1,4-dioxane (1.2 mL) and H₂O (0.15 mL) was heated at 110 ºC in a sealed tube for 3 h. The reaction was partitioned between H₂O and DCM. The aqueous layer was extracted with DCM (x3). The combined organic extract was dried over Na₂SO₄, filtered and 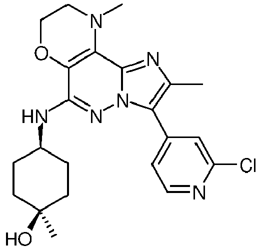 concentrated under vacuum. The residue was purified by column chromatography on silica gel using a solvent gradient from 0% to 5% of MeOH in DCM to render Compound Nr. **28** (solid, 17 mg; 34%). LCMS (ESI): Rt = 3.87 min, m/z = 443.20 = [M+H]⁺. ¹H NMR (300 MHz, DMSO-d6) δ ppm 8.41 (d, *J* = 5.4 Hz, 1H), 8.18 (d, *J* = 1.5 Hz, 1H), 7.69 (dd, *J* = 5.4, 1.5 Hz, 1H), 5.72 (d, *J* = 7.4 Hz, 1H), 4.40 - 4.14 (m, 2H), 4.04 (s, 1H), 3.51 (s, 3H), 3.38 (m, 2H, under water signal), 2.50 (3H under dmso signal), 2.49 - 2.43 (m, 1H), 1.80 - 1.51 (m, 6H), 1.48 - 1.34 (m, 2H), 1.12 (s, 3H).

### Example 18. Compound Nr. 29: 4-[2,9-Dimethyl-3-(2-trifluoromethyl-pyridin-4-yl)-8,9-dihydro-7H-6-oxa-1,3a,4,9-tetraaza-cyclopenta[a]naphthalen-5-ylamino]-1-methyl-cyclohexanol

A mixture of **Intermediate LXV** (12 mg, 0.03 mmol), cis-4-amino-1-methyl-cyclohexanol (6 mg, 0.05 mmol), Na^{t}BuO (10 mg, 0.06 mmol), Xantphos (5 mg, 0.002 mmol) and Pd₂(dba)₃ (5 mg) in dioxane (0.5 mL) was heated under microwave irradiation at 100 ºC for 1h. The dark mixture was filtered through a Celite pad, rinsing with DCM, and the filtrate was concentrated under vacuum. The crude was purified by column chromatography on silica gel using a solvent gradient first from 50% to 100% of EtOAc in cHex 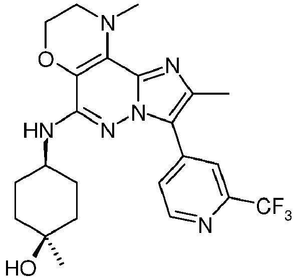 and after from 0% to 5% of MeOH in EtOAc. The obtained product was triturated with methanol, to give required Compound Nr. **29** (crystal white solid, 5 mg, 33%). LCMS (ESI): Rt =4.02 min, m/z = 477.20 [M+H]⁺. ¹H NMR (300 MHz, DMSO-d6) δ ppm 8.76 (d, *J*= 4.9 Hz, 1H), 8.57 (s, 1H), 7.91 (d, *J* = 4.7 Hz, 1H), 7.53 (br s, 1H), 5.71 (d, *J* = 7.6 Hz, 1H), 4.28 (d, *J* = 2.8 Hz, 2H), 4.02 (s, 1H), 3.52 (s, 3H), 3.39 (d, *J* = 3.0 Hz, 2H), 2.44 (3H,under dmso signal), 1.77 - 1.63 (m, 4H), 1.62 - 1.53 (m, 2H), 1.38 - 1.29 (m, 2H), 1.10 (s, 3H).

### Example 19. Compound Nr. 33: [3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine

Intermediate **LIV** (80 mg, 0.151 mmol) in DCM (3 mL) was treated with TFA (0.29 mL, 3.775 mmol) and the mixture was stirred at rt for 4 h. The mixture was concentrated under vacuum. The residue was purified by column chromatography on SCX-2 cartridge using a solvent gradient from 0% to 5% of NH₃ (7N in MeOH) in MeOH and then by HPLC to render Compound Nr. **33** as formic acid salt (white solid, 24 mg, 37%). 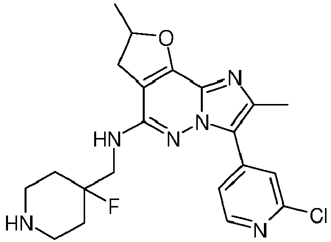 LCMS (ESI): Rt = 5.00 min, m/z = 431.10 = [M+H]⁺. ¹H NMR (300 MHz, DMSO-*d6*) δ ppm 8.48 (d, *J* = 5.3 Hz, 1H), 8.33 (s, 1H), 8.13 (s, 1H), 7.78 (dd, *J* = 5.3, 1.3 Hz, 1H), 6.83 (s, 1H), 5.31 (dd, *J* = 15.4, 6.6 Hz, 1H), 3.60 (2H under water signal), 3.34 (dd, *J* = 15.6, 9.6 Hz, 1H), 2.99 (m, 2H), 2.90 - 2.64 (m, 3H), 2.50 (s, 3H under dmso signal), 1.85 (m, 3H), 1.70 (m, 1H), 1.49 (d, *J* = 6.2 Hz, 3H).

### Example 20, Example 21. Compounds Nr. 34 and 35:

### [(R)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine and [(S)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine.

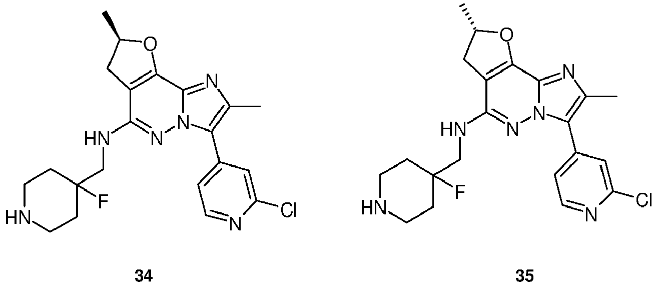

Racemic Product **33** was separated in its enantiomers by chiral prep-HPLC: Chiral IC; Hept/EtOH/EDA: 70/30/0.1; 2 mL/min; 50 min; 230 nm. First compound eluted with Rt1= 37.184 min. It was collected and concentrated under vacuum; then, EDA was removed by column chromatography on silica gel eluting with DCM-MeOH-NH₃ (7N in MeOH) 9:1:0.1 to render Compound Nr. **34** (white solid, 4mg, random assignation as enantiomer *R*). The second compound eluted at Rt2= 45.176 min. It was collected and concentrated under vacuum and then, EDA was removed by column chromatography on silica gel eluting with DCM-MeOH 7:3 to yield Compound Nr. **35** (off-white solid, 5mg, random assignation as enantiomer S).

Compound Nr. **34:** 95% ee; Chiral IC, Hept/EtOH/EDA 70/30/0.1; 0.8 mL/min; 30 min; Rt = 21.40min. LCMS (ESI): Rt = 5.00 min, m/z = 431.10 = [M+H]⁺. ¹H NMR (300 MHz, DMSO-*d6*) δ ppm 8.47 (d, *J* = 5.3 Hz, 1H), 8.17 (d, *J* = 0.9 Hz, 1H), 7.76 (dd, *J* = 5.4, 1.5 Hz, 1H), 6.74 (t, *J* = 6.2 Hz, 1H), 5.40 - 5.22 (m, 1H), 3.57 (d, *J* = 6.3 Hz, 1H), 3.50 (d, *J* = 6.3 Hz, 1H), 2.50 (s, 3H under dmso signal), 2.82 - 2.61 (m, 5H), 1.76 - 1.51 (m, 4H), 1.49 (d, *J* = 6.3 Hz, 3H).

Compound Nr. **35:** 95% ee; Chiral IC, Hept/EtOH/EDA 70/30/0.1; 0.8 mL/min; 30 min; 230 nm, Rt = 26.10 min. LCMS (ESI): Rt = 5.00 min, m/z = 431.10 = [M+H]⁺. ¹H NMR (300 MHz, DMSO-*d6*) δ ppm 8.47 (d, *J* = 5.3 Hz, 1H), 8.17 (d, *J* = 0.9 Hz, 1H), 7.76 (dd, *J* = 5.4, 1.5 Hz, 1H), 6.74 (t, *J* = 6.2 Hz, 1H), 5.39 - 5.24 (m, 1H), 3.57 (d, *J* = 6.4 Hz, 1H), 3.50 (d, *J* = 6.5 Hz, 1H), 2.50 (s, 3H under dmso signal), 2.82 - 2.61 (m, 5H), 1.62 (m, 4H), 1.49 (d, *J* = 6.3 Hz, 3H).

### Synthesis of intermediates

### 8-Allyloxy-6-chloro-2-methyl-imidazo[1,2-b]pyridazine, Intermediate I

8-Bromo-6-chloro-2-methyl-imidazo[1,2-b]pyridazine (4.6 g, 18.66 mmol) in acetonitrile (62 mL), allyl alcohol (1.9 mL, 27.99 mmol) and Cs₂CO₃ (9.1 g, 27.99 mmol) were heated at 75 ºC in pressure tube over the weekend. More allyl alcohol (0.6 mL) and Cs₂CO₃ (3 g) were added and mixture was further stirred and heated for 3 h. Ethyl acetate and water were added. The layers were separated and the aqueous phase was 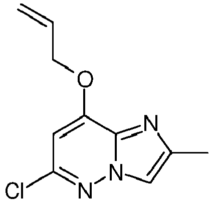 extracted twice with EtOAc. The combined organic extract was dried (Na₂SO₄) and concentrated under vacuum. The crude product was purified by column chromatography on silica gel using a solvent gradient from 0% to 30% of EtOAc in cHex affording Intermediate **I** (white solid, 2.36 g, 56%). LCMS (ESI): Rt = 2.91 min, m/z = 224.10 = [M+H]⁺. ¹H NMR (300 MHz, DMSO-*d6*) δ ppm 7.98 (d, *J* = 0.8 Hz, 1H), 6.88 (s, 1H), 6.20 - 5.98 (m, 1H), 5.50 (dq, *J* = 17.2, 1.6 Hz, 1H), 5.37 (ddd, *J* = 10.5, 2.8, 1.2 Hz, 1H), 4.90 (dt, *J* = 5.5, 1.3 Hz, 2H), 2.33 (d, *J* = 0.7 Hz, 3H).

### 7-Allyl-6-chloro-2-methyl-imidazo[1,2-b]pyridazin-8-ol, Intermediate II

Intermediate **I** (2.36 g, 10.55 mmol) in *t*-BuOH (42 mL) was heated in pressure tube at 175 ºC for 48 h. The solid in suspension was filtered and washed with diethyl ether rendering first batch of desired product as a white solid. The filtrate was concentrated and re-dissolved in *t-* 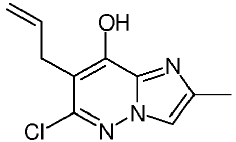 BuOH and heated again for 24 h. A new batch of product was got. The process was repeated again to render Intermediate **II** (white solid, 2.11 g, 89%). LCMS (ESI): Rt = 3.20 min, m/z = 224.10 = [M+H]⁺. ¹H NMR (300 MHz, DMSO-d6) δ ppm 7.82 (s, 1H), 5.94 - 5.70 (m, 1H), 4.96 (dd, *J*= 13.6, 5.3 Hz, 2H), 3.34 (d, *J* = 5.9 Hz, 2H), 2.34 (s, 3H).

### 6-Chloro-7-(3-hydroxy-propyl)-2-methyl-imidazo[1,2-b]pyridazin-8-ol, Intermediate III

To intermediate **II** (1.12 g, 5.0 mmol) in THF (34 mL) at 0 ºC was added borane dimethylsulfide complex (2M in THF, 10 mL, 20 mmol) and the mixture was stirred and allowed to reach rt slowly overnight. At 0 ºC the mixture was carefully quenched with water (25 mL) and when bubbling finished, sodium perborate (5 eq, 4.5 g) was added and the mixture 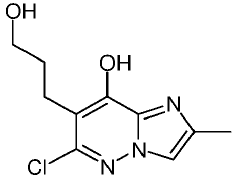 stirred for 3 h. Then, it was acidified with HCl 1N (pH = 2) and then extracted with DCM. The organic layer was filtered to remove inorganic salts and then dried (Na₂SO₄) and concentrated under vacuum rendering Intermediate **III** (white solid, 500 mg, 41% mixture of Intermediate **III** and 6-Chloro-7-(2-hydroxy-propyl)-2-methyl-imidazo[1,2-b]pyridazin-8-ol) which was used in next reaction step without further purification. LCMS (ESI): Rt = 1.65-1.78 min, m/z = 242.10 = [M+H]⁺ and 1.99min -2.16min, m/z = 242.10 = [M+H]⁺.

### 5-Chloro-2-methyl-7,8-dihydro-6H-9-oxa-1,3a,4-triaza-cyclopenta[a]naphthalene, Intermediate IV

The crude Intermediate **III** (500 mg, 2.07 mmol) was suspended in DCM (20 mL) and then Vilsmaier reagent (291 mg, 2.277 mmol) was added and later Et₃N (1.15 mL, 8.28 mmol). After 2 h, water was added to the mixture and it was extracted with DCM. The organic extract was dried and concentrated. The residue was purified by column chromatography on 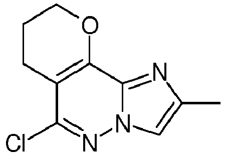 silica gel using a solvent gradient from 50% to 100% of EtOAc in cHex to render Intermediate **IV** (white solid, 140 mg, 30%). LCMS (ESI): Rt = 0.91 min, m/z = 224.10 = [M+H]⁺. ¹H NMR (300 MHz, CDCl₃) δ ppm 7.55 (d, *J* = 0.7 Hz, 1H), 4.58 - 4.37 (m, 2H), 2.76 (t, *J* = 6.4 Hz, 2H), 2.44 (d, *J* = 0.8 Hz, 3H), 2.23 - 2.09 (m, 2H).

### 4-Fluoro-4-[(2-methyl-7,8-dihydro-6H-9-oxa-1,3a,4-triaza-cyclopenta[a]naphthalen-5-ylamino)-methyl]-piperidine-1-carboxylic acid tert-butyl ester, Intermediate V

Intermediate **IV** (114 mg, 0.51 mmol), BINAP (32 mg, 0.051 mmol), tert-butyl-4-(aminomethyl)-4-fluoropiperidine-1-carboxylate (178 mg, 0.765 mmol), NaO^{t}Bu (98 mg, 1.02 mmol) and Pd₂(dba)₃ (47 mg, 0.051 mmol) were mixed in pressure tube and suspended in dioxane (5 mL). The mixture was bubbled with Argon for few minutes and then heated at 110 ºC for 2 h. Ethyl 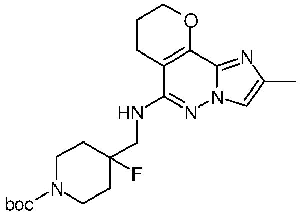 acetate and water were added and layers separated. The organic phase was dried and concentrated. Purification by column chromatography on silica gel using a solvent gradient from 0% to 35% of EtOAc in cHex and then with 0% to 15% of MeOH in EtOAc afforded Intermediate **V** (syrup, 240 mg, 95%). LCMS (ESI): Rt = 3.35 min, m/z = 420.20 = [M+H]⁺.

### 4-[(3-Bromo-2-methyl-7,8-dihydro-6H-9-oxa-1,3a,4-triaza-cyclopenta[a]naphthalen-5-ylamino)-methyl]-4-fluoro-piperidine-1-carboxylic acid tert-butyl ester, Intermediate VI

Intermediate **V** (245 mg, 0.584 mmol) in chloroform (6 mL) was treated with NBS (109 mg, 0.613 mmol) and stirred at rt for 1 h. The reaction mixture was taken in DCM and washed with NaHCO₃ (sat sol). The organic phase was dried (MgSO₄) and concentrated. The residue was purified by column chromatography on silica gel using a solvent gradient from 0% to 100% of EtOAc in cHex to 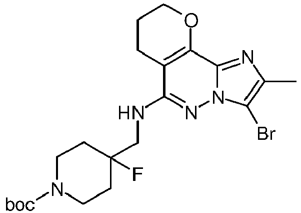 yield Intermediate **VI** (yellowish syrup, 240 mg, 82%). LCMS (ESI): Rt = 3.91 min, m/z = 498.20/500.20 = [M+H]⁺.

### 4-{[3-(2-Chloro-pyridin-4-yl)-2-methyl-7,8-dihydro-6H-9-oxa-1,3a,4-triaza-cyclopenta[a]naphthalen-5-ylamino]-methyl}-4-fluoro-piperidine-1-carboxylic acid tert-butyl ester, Intermediate VII

Intermediate **VI** (100 mg, 0.201 mmol), 2-chloropyridine-4-boronic acid (47 mg, 0.302 mmol), Cs₂CO₃ (131 mg, 0.402 mmol) and PdCl₂dppf (16 mg, 0.020 mmol) were mixed in dioxane (2 mL) and water (0.25 mL) and heated in pressure tube at 120 ºC for 3 h. The mixture was diluted with ethyl acetate and water was added. The layers were separated 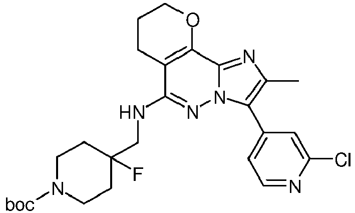 and the aqueous phase was extracted with ethyl acetate. The combined organic extract was dried and concentrated. Purification by column chromatography on silica gel using a solvent gradient from 50% to 100% of EtOAc in cHex afforded Intermediate **VII** (syrup, 44 mg, 41%). %). LCMS (ESI): Rt = 3.87 min, m/z = 531.20 = [M+H]⁺.

### [1-(2-Methyl-5a,7,8,9a-tetrahydro-6H-9-oxa-1,3a,4-triaza-cyclopenta[a]naphthalen-5-yl)-piperidin-3-ylmethyl]-carbamic acid tert-butyl ester, Intermediate VIII

Intermediate **IV** (100 mg, 0.447 mmol), 3-(boc-aminomethyl)piperidine (144 mg, 0.671 mmol), BINAP (28 mg, 0.045 mmol), NaO^{t}Bu (86 mg, 0.894 mmol) and Pd₂(dba)₃ (41 mg, 0.045 mmol) were suspended in dioxane (4 mL) in pressure tube. The mixture was purged with Argon for few minutes and then heated at 110 ºC for 7 h. The mixture was taken in ethyl acetate and water. The layers were separated. The aqueous phase was extracted with EtOAc and the combined organic 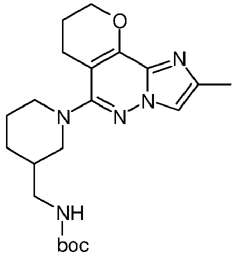 extract was dried (MgSO₄) and concentrated. The crude product was purified by column chromatography on silica gel using a solvent gradient from 20% to 100% of EtOAc in cHex affording Intermediate **VIII** (63mg, 35%). LCMS (ESI): Rt = 3.37 min, m/z = 402.10 = [M+H]⁺.

### [1-(3-Bromo-2-methyl-7,8-dihydro-6H-9-oxa-1,3a,4-triaza-cyclopenta[a]naphthalen-5-yl)-piperidin-3-ylmethyl]-carbamic acid tert-butyl ester, Intermediate IX

To intermediate **VIII** (60 mg, 1 eq, 0.149 mmol) in chloroform (2 mL) was added NBS (27 mg, 0.149 mmol). The mixture was stirred at rt for 90 min. NaHCO₃ (sat sol) was added to the mixture and it was extracted with DCM. The organic phase was washed with brine, dried (MgSO₄) and concentrated. The residue was purified by column chromatography on silica gel using a solvent gradient from 20% to 100% of EtOAc in cHex rendering Intermediate **IX** (42 mg, 58%). LCMS (ESI): Rt = 4.20 min, m/z = 480.20/482.20 = [M+H]⁺.

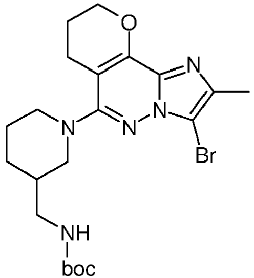

### {1-[3-(2-Chloro-pyridin-4-yl)-2-methyl-7,8-dihydro-6H-9-oxa-1,3a,4-triaza-cyclopenta[a]naphthalen-5-yl]-piperidin-3-ylmethyl}-carbamic acid tert-butyl ester, Intermediate X

Intermediate **IX** (42 mg, 0.087 mmol), 2-chloropyridine-4-boronic acid (1.5 eq, 21 mg, 0.131 mmol), PdCl₂dppf (7 mg, 0.009 mmol) and Cs₂CO₃ (57 mg, 0.174 mmol) were mixed in dioxane (1 mL) and water (0.1 mL). The mixture in pressure tube was heated at 110 ºC for 2 h. The mixture was taken in ethyl acetate and water. The layers were separated. The aqueous phase was extracted with ethyl acetate. The combined organic extract was dried 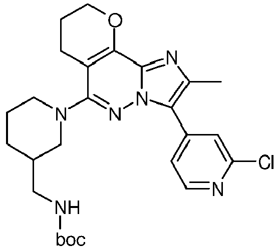 (MgSO₄) and concentrated. The crude product was purified by column chromatography on silica gel using a solvent gradient from 10% to 100% of EtOAc in cHex to yield Intermediate **X** (syrup, 25 mg, 56%). LCMS (ESI): Rt = 4.22 min, m/z = 513.20 = [M+H]⁺.

### [(S)-1-(2-Methyl-7,8-dihydro-6H-9-oxa-1,3a,4-triaza-cyclopenta[a]naphthalen-5-yl)-piperidin-3-ylmethyl]-carbamic acid tert-butyl ester, Intermediate XI

Intermediate **IV** (288 mg, 1.288 mmol), (R)-3-(boc-aminomethyl)-piperidine (469 mg, 2.190 mmol), NaO^{t}Bu (247mg, 2.576 mmol), X-Phos (92mg, 0.193 mmol) and Pd₂dba₃ (118mg, 0.129 mmol) were suspended in dioxane (13 mL) and Ar flow was passed through the mixture for 5min before heating in pressure tube at 115 ºC for 2 h. The reaction mixture was taken in ethyl acetate and washed with water. The organic phase was dried and concentrated. The crude product was purified by column chromatography on silica gel using 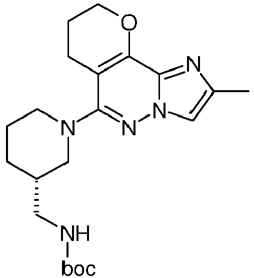 a solvent gradient from 20% to 100% of EtOAc in cHex to yield Intermediate **XI** (syrup, 266 mg, 51%). LCMS (ESI): Rt = 3.45 min, m/z = 402.20 = [M+H]⁺.

### [(S)-1-(3-Bromo-2-methyl-7,8-dihydro-6H-9-oxa-1,3a,4-triaza-cyclopenta[a]naphthalen-5-yl)-piperidin-3-ylmethyl]-carbamic acid tert-butyl ester, Intermediate XII

To intermediate **XI** (260 mg, 0.648 mmol) in CHCl₃ (7mL) was added NBS (86 mg, 0.486 mmol). The reaction mixture was stirred at rt for 20 min. The mixture was taken in DCM and washed with NaHCO₃ (sat sol) and water. The organic phase was dried (Na₂SO₄) and concentrated. The crude product was purified by column chromatography on silica gel using a solvent gradient from 20% to 100% of EtOAc in cHex and a second column with gradient from 0% to 10% of MeOH in DCM to render Intermediate **XII** (yellow solid, 160 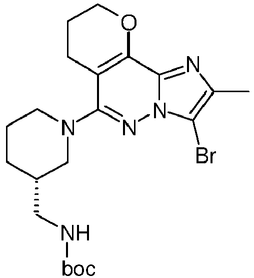 mg, 51%). LCMS (ESI): Rt = 4.38 min, m/z = 480.20/482.20 = [M+H]⁺.

### {(S)-1-[3-(2-Chloro-pyridin-4-yl)-2-methyl-7,8-dihydro-6H-9-oxa-1,3a,4-triaza-cyclopenta[a]naphthalen-5-yl]-piperidin-3-ylmethyl}-carbamic acid tert-butyl ester, Intermediate XIII

Intermediate **XII** (155 mg, 0.323 mmol), 2-chloropyridine-4-boronic acid (76 mg, 0.485 mmol), PdCl₂dppf (26 mg, 0.032 mmol) and Cs₂CO₃ (210 mg, 0.646 mmol) were mixed in dioxane (3 mL) and water (0.4 mL). The mixture in pressure tube was heated at 115 ºC for 3 h. The mixture was taken in ethyl acetate and water. The layers were separated. The aqueous phase was extracted with ethyl acetate. The combined organic extract was 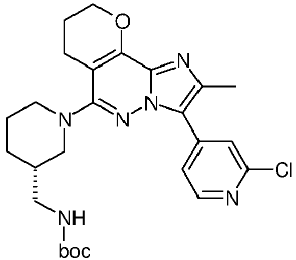 dried (MgSO₄) and concentrated. The crude product was purified by column chromatography on silica gel using a solvent gradient from 20% to 100% of EtOAc in cHex yielding Intermediate **XIII** (syrup, 92 mg, 55%). LCMS (ESI): Rt = 4.22 min, m/z = 513.10 = [M+H]⁺.

### [(R)-1-(2-Methyl-7,8-dihydro-6H-9-oxa-1,3a,4-triaza-cyclopenta[a]naphthalen-5-yl)-piperidin-3-ylmethyl]-carbamic acid tert-butyl ester, Intermediate XIV

Intermediate **IV** (150 mg, 0.671 mmol), (S)-3-(boc-aminomethyl)-piperidine (244 mg, 1.141 mmol), X-Phos (16 mg, 0.134 mmol), NaO^{t}Bu (61 mg, 1.342 mmol) and Pd₂(dba)₃(64 mg, 0.067 mmol) were mixed in pressure tube and suspended in dioxane (7 mL). The mixture was bubbled with Argon for few minutes and then heated at 100 ºC for 90 min under microwave irradiation. The reaction mixture was taken in ethyl acetate and water. Layers were separated and the aqueous phase was extracted with ethyl acetate. The combined organic layer was dried and concentrated. The crude product was purified by column chromatography on silica gel using a solvent gradient from 20% to 100% of EtOAc in cHex rendering Intermediate **XIV** (syrup, 95 mg, 35%). LCMS (ESI): Rt = 3.34 min, m/z = 402.20 = [M+H]⁺.

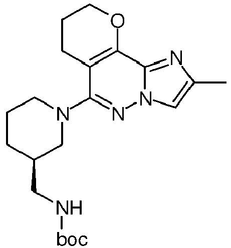

### [(R)-1-(3-Bromo-2-methyl-7,8-dihydro-6H-9-oxa-1,3a,4-triaza-cyclopenta[a] naphthalen-5-yl)-piperidin-3-ylmethyl]-carbamic acid tert-butyl ester, Intermediate XV

Intermediate **XIV** (80 mg, 0.20 mmol) in CHCl₃ (3 mL) was treated with NBS (28 mg, 0.16 mmol). The mixture was stirred at rt for 45 min. The reaction mixture was taken in DCM and water. Layers were separated. The organic layer was dried (MgSO₄) and concentrated to render Intermediate **XV** which was used in next reaction step without further purification (assumed 100%). LCMS (ESI): Rt = 4.32 min, m/z = 480.20/482.20 = [M+H]⁺.

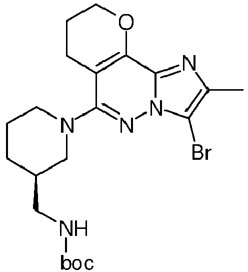

### {(R)-1-[3-(2-Chloro-pyridin-4-yl)-2-methyl-7,8-dihydro-6H-9-oxa-1,3a,4-triaza-cyclopenta[a]naphthalen-5-yl]-piperidin-3-ylmethyl}-carbamic acid tert-butyl ester, Intermediate XVI

Intermediate **XV** (96 mg, 0.200 mmol), 2-chloropyridine-4-boronic acid (47 mg, 0.300 mmol), PdCl₂dppf (16 mg, 0.02 mmol) and Cs₂CO₃ (130 mg, 0.400 mmol) were mixed in dioxane (2 mL) and water (0.2 mL). The mixture in pressure tube was heated at 110 ºC for 20 h. The mixture was taken in ethyl acetate and water. The layers were separated. The aqueous phase was extracted with ethyl acetate. The combined organic extract was dried (MgSO₄) and concentrated. The crude product was purified by column chromatography on silica gel using a solvent gradient from 20% to 100% of EtOAc in cHex yielding Intermediate **XVI** (syrup, 71 mg, 69%). LCMS (ESI): Rt = 4.22 min, m/z = 513.20 = [M+H]⁺.

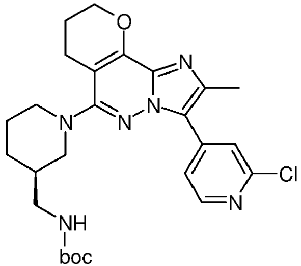

### 1-Methyl-4-(2-methyl-7,8-dihydro-6H-9-oxa-1,3a,4-triaza-cyclopenta[a]naphthalen-5-ylamino)-cyclohexanol, Intermediate XVII

Intermediate **IV** (40 mg, 0.179 mmol), cis-4-amino-1-methyl-cyclohexanol (28 mg, 0.215 mmol), BINAP (10 mg, 0.018mmol), NaO^{t}Bu (34 mg, 0.358 mmol) and Pd(dba)₂ (10 mg, 0.018 mmol) were mixed in pressure tube in dioxane (2 mL). The mixture was purged with Argon for few minutes and then heated at 110 ºC overnight. Excess of reagents were added to complete the reaction. The mixture was filtered through Celite pad and the filtrate was concentrated. The residue was purified by 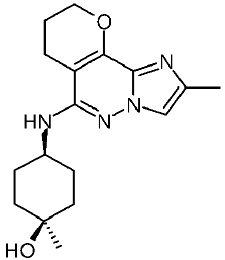 column chromatography on silica gel using a solvent gradient from 50% to 100% of EtOAc in cHex and then with gradient from 0% to 15% of MeOH in EtOAc to render Intermediate **XVII** (syrup, 32 mg, 57%). LCMS (ESI): Rt = 0.65 min, m/z = 317.20 = [M+H]⁺.

### 4-(3-Bromo-2-methyl-7,8-dihydro-6H-9-oxa-1,3a,4-triaza-cyclopenta[a]naphthalen-5-ylamino)-1-methyl-cyclohexanol, Intermediate XVIII

To intermediate **XVII** (32 mg, 0.101 mmol) in CHCl₃ (1mL) was added NBS (18 mg, 0.103 mmol) and the mixture was stirred at rt for 90 min. The mixture was taken in DCM and water. Layers were separated and the organic layer was washed with NaHCO₃ (sat sol), dried (MgSO₄) and concentrated to render Intermediate **XVIII** (assuming quantitative yield) which was used in next reaction step without further purification. LCMS (ESI): Rt = 3.22 min, m/z = 395.20/397.20 = [M+H]⁺.

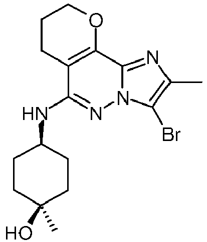

### 4-Fluoro-4-[(2-methyl-3-pyridin-4-yl-7,8-dihydro-6H-9-oxa-1,3a,4-triaza-cyclopenta[a]naphthalen-5-ylamino)-methyl]-piperidine-1-carboxylic acid tert-butyl ester, Intermediate XIX

Intermediate **VI** (100 mg, 0.201 mmol), pyridine-4-boronic acid pinacol ester (62 mg, 0.302 mmol), Cs₂CO₃ (131 mg, 0.402 mmol) and PdCl₂dppf (16 mg, 0.020 mmol) were mixed in dioxane (2 mL) and water (0.25 mL) and heated in pressure tube at 120 ºC for 1 h. Excess of reagents were added till completion of the reaction. The mixture was taken in EtOAc 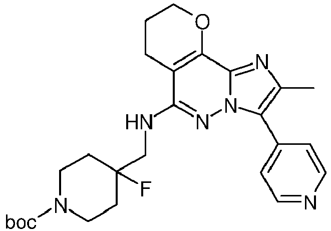 and water. Layers were separated. The aqueous phase was extracted twice with ethyl acetate. The combined organic extract was dried and concentrated. Purification by column chromatography on silica gel using a solvent gradient from 0% to 10% of MeOH in EtOAc rendered Intermediate **XIX** (syrup, 45 mg, 45%). LCMS (ESI): Rt = 3.21 min, m/z = 497.20 = [M+H]⁺.

### 5-Chloro-2,7-dimethyl-6,7-dihydro-8-oxa-1,3a,4-triaza-as-indacene, Intermediate XXI

Intermediate **II** (630 mg, 2.817 mmol) in 1,2-DCE (20 mL) under Ar atmosphere was treated with TiCl₄ (1M in DCM, 8.45 mL, 8.451 mmol) and the mixture in pressure tube was heated at 75 ºC for 48 h. Excess of TiCl₄ was added till completion of the reaction after 80 h of heating. Water was added to the mixture and then NaHCO₃ (sat sol). Layers were separated. 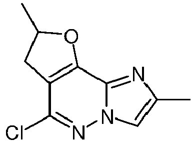 The aqueous phase was extracted with DCM. The organic extract was dried and concentrated. The crude product was triturated with DCM and the filtrate was purified by column chromatography on silica gel using a solvent gradient from 50% to 100% of EtOAc in cHex affording Intermediate **XXI** (orange gummy solid, 296 mg, 47%). LCMS (ESI): Rt = 3.00 min, m/z = 224.10 = [M+H]⁺. ¹H NMR (300 MHz, CDCl₃) δ ppm 7.67 (s, 1H), 5.39 (m, *J* = 9.4, 6.4 Hz, 1H), 3.50 (dd, *J* = 15.5, 9.5 Hz, 1H), 3.04 - 2.89 (m, 1H), 2.50 (s, 3H), 1.62 (d, J = 6.3 Hz, 3H).

### 4-(2,7-Dimethyl-6,7-dihydro-8-oxa-1,3a,4-triaza-as-indacen-5-ylamino)-1-methyl-cyclohexanol, Intermediate XXII

Intermediate **XXI** (37 mg, 0.165 mmol), cis-4-amino-1-methyl-cyclohexanol (32 mg, 0.248 mmol), BINAP (10 mg, 0.017 mmol), NaO^{t}Bu (32 mg, 0.330 mmol) and Pd₂(dba)₃ (15 mg, 0.017 mmol) were mixed in pressure tube and suspended in dioxane (2 mL). The mixture was purged with Ar for few minutes and then heated at 110 ºC for 2 h. The mixture was taken in EtOAc and water. Layers were separated. The organic phase was dried and concentrated. The crude product was purified by 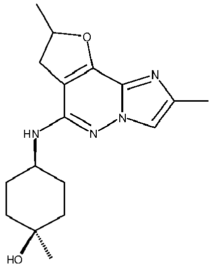 column chromatography on silica gel using a solvent gradient from 50% to 100% of EtOAc in cHex affording Intermediate **XXII** (40 mg, 76%). LCMS (ESI): Rt = 2.48 min, m/z = 317.20 = [M+H]⁺.

### 4-(3-Bromo-2,7-dimethyl-6,7-dihydro-8-oxa-1,3a,4-triaza-as-indacen-5-ylamino)-1-methyl-cyclohexanol, Intermediate XXIII

Intermediate **XXII** (20 mg, 0.126 mmol) in CHCl₃ (2 mL) was treated with NBS (24 mg, 0.132 mmol) and the mixture was stirred at rt for 2 h. The mixture was diluted with DCM and washed with NaHCO₃ (sat sol). The organic layer was dried and concentrated to render Intermediate **XXIII** (50 mg, 100%) that was used without further purification in next step. LCMS (ESI): Rt = 3.68 min, m/z = 395.20/397.20 = [M+H]⁺.

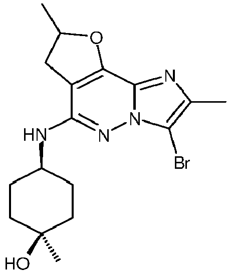

### 5-Chloro-4-methyl-3,4-dihydro-2H-pyridazino[4,5-b][1,4]oxazin-8-ylamine/8-Chloro-4-methyl-3,4-dihydro-2H-pyridazino[4,5-b][1,4]oxazin-5-ylamine, Intermediate XIV

A mixture of 5,8-dichloro-4-methyl-3,4-dihydro-2H-pyridazino[4,5-b][1,4]oxazine (4.750 g, 21 mmol) in EtOH (20 mL) and THF (20 mL) with TEA (6 mL,42 mmol) and ammonium hydroxide (35 mL, 845 mmol) was heated at 200 ºC in a Parr reactor for 40 h. Solvents were removed under 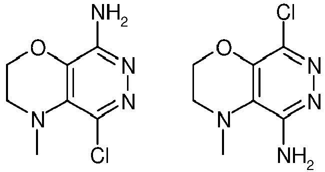 vacuum, and the crude was purified by column chromatography on silica gel using a solvent gradient from 0% to 10% of MeOH in DCM to render Intermediate **XXIV** (1.470 g, 35%) as a mixture of both regioisomers which were used in next reaction step without additional separation. LCMS (ESI): Rt =0.45 min, m/z = 201.00 [M+H]⁺.

### 5-Chloro-2,6-dimethyl-7,8-dihydro-6H-9-oxa-1,3a,4,6-tetraaza-cyclopenta[a] naphthalene, Intermediate XXV-A and 5-Chloro-2,9-dimethyl-8,9-dihydro-7H-6-oxa-1,3a,4,9-tetraaza-cyclopenta[a] naphthale, Intermediate XXV-B

N₂ was bubbled into a mixture of Intermediate **XXIV** (800 mg, 4.0 mmol) with TEA (1.4 mL, 10 mmol), chloroacetone (0.650 ml, 8 mmol) in EtOH (5 mL). The mixture was heated under microwave irradiation at 150 ºC for 30 min. Solvents were removed under vacuum and the crude was taken 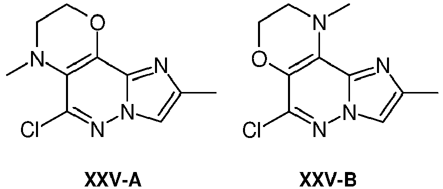 in DCM, washed with water and brine, dried over Na₂SO₄ and concentrated. The crude was purified by column chromatography on silica gel using a solvent gradient from 50% to 100% of EtOAc/cHex to render Intermediate **XXV-B** (first eluting fraction, 290 mg, 15%) and its regioisomer Intermediate **XXV-A** (second eluting fraction, 770 mg, 40%). **XXV-A** LCMS (ESI): Rt =0.6 min, m/z = 239.10 [M+H]⁺.¹H NMR (300 MHz, CDCl₃) δ 7.44 (d, *J* = 0.9 Hz, 1H), 4.37 (dd, *J* = 4.8, 4.0 Hz, 2H), 3.21 - 3.12 (m, 2H), 2.80 (s, 3H), 2.38 (d, *J* = 0.9 Hz, 3H). **XXV-B** LCMS (ESI): Rt=2.34 min, m/z = 239.10 [M+ H]⁺. ¹H NMR (300 MHz, CDCl₃) δ 7.35 (d, *J* = 0.8 Hz, 1H), 4.24 (dd, *J* = 5.8, 3.0 Hz, 2H), 3.74 (s, 3H), 3.43 (dd, *J* = 5.8, 3.0 Hz, 2H), 2.33 (t, *J* = 1.7 Hz, 3H).

### 4-[(2,6-Dimethyl-7,8-dihydro-6H-9-oxa-1,3a,4,6-tetraaza-cyclopenta[a]naphthalen-5-ylamino)-methyl]-piperidine-1-carboxylic acid tert-butyl ester, Intermediate XXVIII

A mixture of **XXV-A** (150 mg, 0.62 mmol), 1-boc-4-(aminomethyl)piperidine (190 mg, 0.88 mmol), NaO^{t}Bu (140 mg, 1.4 mmol), catalytic Pd₂(dba)₃ (35 mg) and BINAP (27 mg) in dioxane (6 mL) was heated in a sealed tube at 110 ºC for 16 h. The dark mixture was concentrated under vacuum, and the crude was purified by column chromatography on silica gel using a solvent gradient from 0% to 10% of MeOH 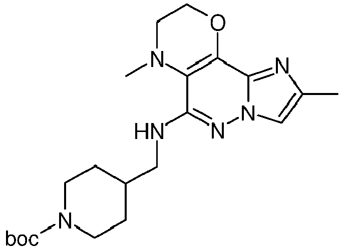 in EtOAc to render Intermediate **XXVIII** (crystal solid, 235 mg, 91%). LCMS (ESI): Rt =3.19 min, m/z = 417.30 [M+H]⁺.¹H NMR (300 MHz, CDCl₃) δ 7.22 (d, *J* = 0.8 Hz, 1H), 4.70 (t, *J* = 5.7 Hz, 1H), 4.37-4.31 (m, 2H), 4.05 (dd, *J* = 14.3, 7.1 Hz, 2H), 3.19 (t, *J* = 6.2 Hz, 2H), 3.10-3.03 (m, 2H), 2.75-2.63 (m, 2H), 2.62 (s, 3H), 2.33 (s, 3H), 1.70 (d, *J* = 13.5 Hz, 2H), 1.39 (s, 9H).

### 4-[(3-Bromo-2,6-dimethyl-7,8-dihydro-6H-9-oxa-1,3a,4,6-tetraaza-cyclopenta[a]naphthalen-5-ylamino)-methyl]-piperidine-1-carboxylic acid tert-butyl ester, Intermediate XXIX

To a solution of Intermediate **XXVIII** (235 mg, 0.56 mmol) in CHCl₃ (3 mL) was added in one pot NBS (110 mg, 0.57 mmol). The resulting mixture was stirred for 30 min. DCM was added, and the organic phase was washed four times with NaHCO₃ (sat sol), twice with water and finally once with brine. The organic layer was dried (Na₂SO₄) and concentrated under vacuum. The crude was triturated with diethyl ether, 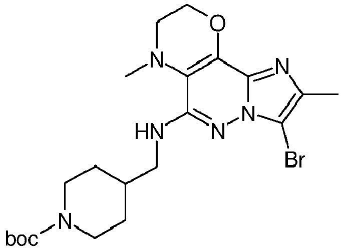 and the filtrate was concentrated under vacuum yielding required Intermediate **XXIX** which was used in next reaction step without further purification (crystal solid, 180 mg, 68%). LCMS (ESI): Rt =3.73 min, m/z = 495.20/497.20 [M+H]⁺.

### 4-{[3-(2-Chloro-pyridin-4-yl)-2,6-dimethyl-7,8-dihydro-6H-9-oxa-1,3a,4,6-tetraaza-cyclopenta[a]naphthalen-5-ylamino]-methyl}-piperidine-1-carboxylic acid tert-butyl ester, Intermediate XXX

A mixture of **XXIX** (60 mg, 0.12 mmol) 2-chloropyridine-4-boronic acid (25 mg, 0.13 mmol), Cs₂CO₃ (100 mg, 0.3 mmol), Pd(dppf)Cl₂ (10 mg) in dioxane (1.2 mL) and water (0.2 mL), was heated 110 ºC for 16 h. The dark mixture was filtered through a Celite pad, rinsing with DCM and some drops of MeOH. The filtrate was concentrated under vacuum, and the residue was 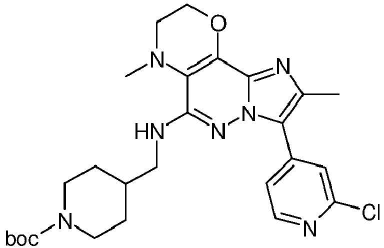 purified by column chromatography on silica gel using a solvent gradient from 0% to 15% of MeOH in EtOAc, yielding the required Intermediate **XXX** (cream solid, 26 mg, 40%). LCMS (ESI): Rt =3.73 min, m/z = 528.30 [M+H]⁺.

### 4-(2,6-Dimethyl-7,8-dihydro-6H-9-oxa-1,3a,4,6-tetraaza-cyclopenta[a]naphthalen-5-ylamino)-butan-1-ol, Intermediate XXXI

A mixture of **XXV-A** (75 mg, 0.3 mmol), 4-amino-1-butanol (35 uL, 0.37 mmol), NaO^{t}Bu (65 mg, 0.66 mmol), catalytic Pd₂(dba)₃ (10 mg) and BINAP (10 mg) in dioxane (3 mL) was heated in a sealed tube at 110 ºC for 3 h. The dark mixture was cooled down and filtered through a Celite Pad 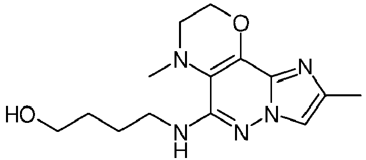 rinsing with DCM. The filtrate was concentrate under vacuum and the residue was purified by column chromatography on silica gel using a solvent gradient from 25% to 100% of EtOAc in cHex and with 10% of MeOH, yielding the product Intermediate **XXXI** (cream solid, 60 mg, 68%). LCMS (ESI): Rt =0.58 min, m/z = 292.20 [M+H]⁺.

### 4-(3-lodo-2,6-dimethyl-7,8-dihydro-6H-9-oxa-1,3a,4,6-tetraaza-cyclopenta[a] naphthalen-5-ylamino)-butan-1-ol, Intermediate XXXII

To a mixture of Intermediate **XXXI** (60 mg, 0.2 mmol) in DMF (1.1 mL) was added NIS (50 mg, 0.2 mmol). The resulting mixture was stirred for 3 h. Water was added to the mixture and it was extracted with EtOAc. The organic phase was washed with NaHCO₃ (sat sol), three times, 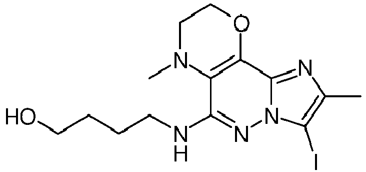 once with water and brine, dried (Na2SO4) and concentrated under vacuum, to yield the Intermediate **XXXII** (mustard solid, 62 mg, 72%) which was used in next reaction step without further purification. LCMS (ESI): Rt =3.04 min, m/z = 418.05 [M+H]⁺.

### 4-(2,6-Dimethyl-7,8-dihydro-6H-9-oxa-1,3a,4,6-tetraaza-cyclopenta[a]naphthalen-5-ylamino)-1-methyl-cyclohexanol, Intermediate XXXIII

A mixture of **XXV-A** (170 mg, 0.72 mmol), cis-4-amino-1-methyl-cyclohexanol (110 mg, 0.8 mmol), NaO^{t}Bu (140 mg, 1.4 mmol), catalytic Pd₂(dba)₃ (30 mg) and BINAP (27 mg) in dioxane (6 mL) was heated in a sealed tube at 110 ºC for 16 h. The dark mixture was concentrated in vacuum, and the crude was purified by column chromatography on silica gel using a solvent gradient from 0% to 10% of MeOH in DCM yielding the Intermediate **XXXIII** (cream solid, 159 mg, 67%). LCMS (ESI): Rt =0.41 min, m/z = 332.20 [M+H]⁺.

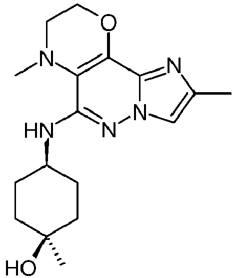

### 4-(3-Iodo-2,6-dimethyl-7,8-dihydro-6H-9-oxa-1,3a,4,6-tetraaza-cyclopenta[a]naphthalen-5-ylamino)-1-methyl-cyclohexanol, Intermediate XXXIV

To a solution of Intermediate **XXXIII** (50 mg, 0.15 mmol) in DMF (1 mL) was added NIS (35 mg, 0.15 mmol). The resulting mixture was stirred for 30 min. The reaction was diluted with water and extracted three times with DCM. The combined organic layers were washed three times with NaHCO₃ (sat sol), once with brine, and water and dried (Na₂SO₄) and concentrated under vacuum to yield the Intermediate **XXXIV** (40 mg, 58%) which was used in next reaction step without further purification. LCMS (ESI): Rt =3.08 min, m/z = 458.10 [M+H]⁺.

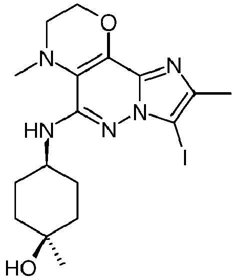

### 4-(2,6-Dimethyl-7,8-dihydro-6H-9-oxa-1,3a,4,6-tetraaza-cyclopenta[a]naphthalen-5-ylamino)-1-methyl-cyclohexanol, Intermediate XXXV

A mixture of **XXV-A** (50 mg, 0.2 mmol), trans-4-amino-1-methyl-cyclohexanol hydrochloride (45 mg, 0.25 mmol), NaO^{t}Bu (40 mg, 0.4 mmol), catalytic Pd₂(dba)₃ (15 mg) and BINAP (7 mg) in dioxane (3 mL) was heated in a sealed tube at 110 ºC for 3 h. The dark mixture was concentrated in vacuum, and the crude was purified by column chromatography on silica gel using a solvent gradient from 0% to 10% of MeOH in DCM, yielding the product Intermediate **XXXV** (brown solid, 60 mg, 86%). LCMS (ESI): Rt =0.49 min, m/z = 332.20 [M+H]⁺.

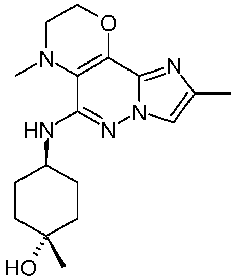

### 4-(3-Iodo-2,6-dimethyl-7,8-dihydro-6H-9-oxa-1,3a,4,6-tetraaza-cyclopenta[a]naphthalen-5-ylamino)-1-methyl-cyclohexanol, Intermediate XXXVI

To a solution of Intermediate **XXXV** (60 mg, 0.18 mmol) in DMF (3 mL) was added NIS (45 mg, 0.19 mmol). The resulting dark mixture was stirred for 1 h. The mixture was diluted with water and extracted with EtOAc. The organic layers was washed three times with NaHCO₃ (sat sol), once with water and brine. After drying the organic phase (Na₂SO₄), the solvents were removed under vacuum. The mustard solid recovered, was purified by column chromatography on silica gel using a solvent gradient from 50% to 100% EtOAc in cHex and 10% of 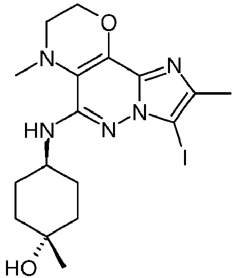 MeOH, to render the product Intermediate **XXXVI** (cream solid, 20 mg, 24%). LCMS (ESI): Rt =2.95 min, m/z = 458.15 [M+H]⁺.

### 4-{[2,6-Dimethyl-3-(2-trifluoromethyl-pyridin-4-yl)-7,8-dihydro-6H-9-oxa-1,3a,4,6-tetraaza-cyclopenta[a]naphthalen-5-ylamino]-methyl}-piperidine-1-carboxylic acid tert-butyl ester, Intermediate XXXVII

A mixture of **XXIX** (60 mg, 0.12 mmol) 2-(trifluoromethyl)pyridine-4-boronic acid (30 mg, 0.13 mmol), Cs₂CO₃ (80 mg, 0.2 mmol), Pd(dppf)Cl₂ (10 mg) in dioxane (1.2 mL) and water (0.2 mL), was heated under microwave irradiation at 150 ºC for 60 min. The dark mixture was filtered through a Celite pad, rinsing with DCM and some drops of MeOH. The filtrate was 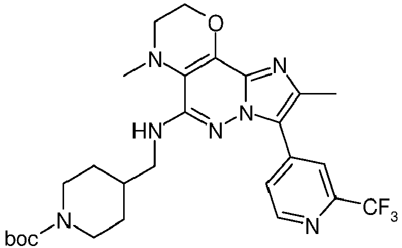 concentrated under vacuum, and the residue was purified by column chromatography on silica gel using a solvent gradient from 0% to 15% of MeOH in EtOAc to yield Intermediate **XXXVII** (white solid, 15 mg, 23%). LCMS (ESI): Rt =3.90 min, m/z = 562.30 [M+H]⁺.

### 4-[(2,6-Dimethyl-3-pyridin-4-yl-7,8-dihydro-6H-9-oxa-1,3a,4,6-tetraaza-cyclopenta [a]naphthalen-5-ylamino)-methyl]-piperidine-1-carboxylic acid tert-butyl ester, Intermediate XXXVIII

A mixture of **XXIX** (60 mg, 0.12 mmol), pyridine-4-boronic acid pinacol ester (30 mg, 0.13 mmol), Cs₂CO₃ (80 mg, 0.2 mmol), Pd(dppf)Cl₂ (10 mg) in dioxane (1.2 mL) and water (0.2 mL), was heated under microwave irradiation at 150 ºC for 60 min. The dark mixture was filtered through a Celite pad, rinsing with DCM and some drops of MeOH. The filtrate was concentrated under vacuum, and the residue was purified by 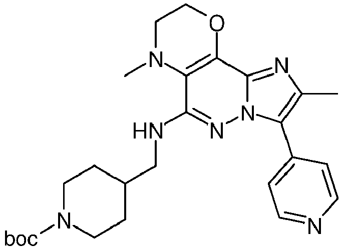 column chromatography on silica gel using a solvent gradient from 0% to 15% MeOH in EtOAc, to render Intermediate **XXXVIII** (white solid, 44 mg, 76%). LCMS (ESI): Rt =3.10 min, m/z = 494.30 [M+H]⁺.

### 4-[(2,9-Dimethyl-8,9-dihydro-7H-6-oxa-1,3a,4,9-tetraaza-cyclopenta[a]naphthalen-5-ylamino)-methyl]-4-fluoro-piperidine-1-carboxylic acid tert-butyl ester, Intermediate XXXIX

A mixture of Intermediate **XXV-B** (210 mg, 0.88 mmol), tert-butyl 4-(aminomethyl)-4-fluoropiperidine-1-carboxylate (245 mg, 1.056 mmol), sodium tert-butoxide (169 mg, 1.76 mmol), Pd₂(dba)₃ (51 mg, 0.088 mmol) and BINAP (33 mg, 0.053 mmol) in dioxane (9 mL) was heated in a sealed tube at 110 ºC for 4 h. The mixture was concentrated and the residue was purified by column chromatography on silica gel using a solvent gradient 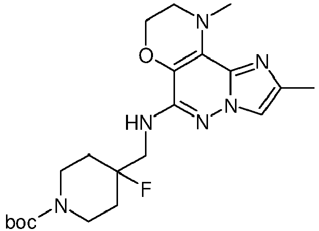 from 0% to 5% of MeOH in DCM to yield Intermediate **XXXIX** (215 mg, 56%). LCMS (ESI): Rt = 3.20 min, m/z = 435.20 = [M+H]⁺.

### 4-[(3-Bromo-2,9-dimethyl-8,9-dihydro-7H-6-oxa-1,3a,4,9-tetraaza-cyclopenta[a]naphthalen-5-ylamino)-methyl]-4-fluoro-piperidine-1-carboxylic acid tert-butyl ester, Intermediate XL

To a solution of Intermediate **XXXIX** (215 mg, 0.495 mmol) in chloroform (5 mL) was added NBS (90 mg, 0.500 mmol). The mixture was stirred at rt for 30 min. Then, it was diluted with DCM and washed twice with NaHCO₃ (sat sol) water and brine. The organic layer was dried and concentrated. The crude product was purified by column chromatography on silica gel using a solvent gradient from 0% to 40% of EtOAc in cHex to 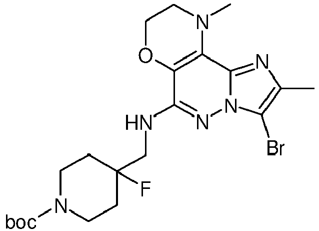 render Intermediate **XL** (85 mg; 34%). LCMS (ESI): Rt = 4.30 min, m/z = 513.20/515.20 = [M+H]⁺.

### 4-{[3-(2-Chloro-pyridin-4-yl)-2,9-dimethyl-8,9-dihydro-7H-6-oxa-1,3a,4,9-tetraaza-cyclopenta[a]naphthalen-5-ylamino]-methyl}-4-fluoro-piperidine-1-carboxylic acid tert-butyl ester, Intermediate XLI

A mixture of Intermediate **XL** (84 mg, 0.165 mmol), 2-chloropyridine-4-boronic acid (39 mg, 0.248 mmol), Pd(dppf)Cl₂ (13 mg, 0.017 mmol) and Cs₂CO₃ (107 mg, 0.330 mmol) in 1,4-dioxane (2 mL) and H₂O (0.2 mL) was heated at 100 ºC in a sealed tube for 5h. The reaction was partitioned between H₂O and DCM. The aqueous layer was extracted with DCM. The combined organic layers 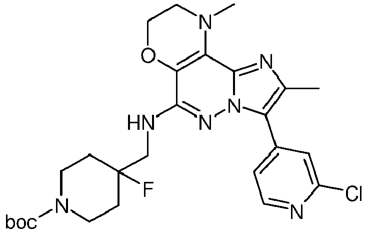 were dried over Na₂SO₄, filtered and concentrated. The residue was purified by column chromatography on silica gel using a solvent gradient from 0% to 3% of MeOH in DCM to render Intermediate **XLI** (46 mg, 51%). LCMS (ESI): Rt = 4.10 min, m/z = 546.20 = [M+H]⁺.

### 3-Bromo-5-chloro-2,9-dimethyl-8,9-dihydro-7H-6-oxa-1,3a,4,9-tetraaza-cyclopenta[a]naphthalene, Intermediate XLII

To a solution of **XXV-B** (290 mg, 1.2 mmol) in CHCl₃ (5 mL) was added in one pot NBS (220 mg, 1.22 mmol). The resulting mixture was stirred for 30 min diluted with DCM and washed several times with NaHCO₃ (sat sol), twice with water and brine. The organic layer was dried (Na₂SO₄) and concentrated under vacuum to render Intermediate **XLII** which was used 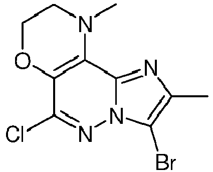 in next reaction step without further purification (cream solid, 390 mg, assumed 100%). LCMS (ESI): Rt =4.39 min, m/z = 317.00/319.00 [M+H]⁺. NMR (300 MHz, CDCl₃) δ 4.29 - 4.22 (m, 2H), 3.73 (s, 3H), 3.46 - 3.40 (m, 2H), 2.33 (s, 3H).

### 5-Chloro-2,9-dimethyl-3-pyridin-4-yl-8,9-dihydro-7H-6-oxa-1,3a,4,9-tetraaza-cyclopenta[a]naphthalene, Intermediate XLIII

A mixture of Intermediate **XLII** (0.754 mmol), pyridine-4-boronic acid pinacol ester (178 mg, 0.867mmol), Pd(dppf)Cl₂ (47 mg, 0.057 mmol) and Cs₂CO₃ (491 mg, 1.508 mmol) in dioxane:H₂O (3 mL: 1 mL) was heated at 100 ºC for 4 h. DCM and water were added. The organic phase was separated, dried (Na₂SO₄), filtered and evaporated under vacuum. The residue was purified by column chromatography on silica gel using a solvent system of 4% MeOH in DCM to Intermediate **XLIII** (30 mg, 12%). LCMS (ESI): Rt =2.39 min, m/z = 316.00 [M+H]⁺.

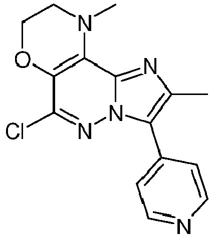

### 4-(2,9-Dimethyl-8,9-dihydro-7H-6-oxa-1,3a,4,9-tetraaza-cyclopenta[a]naphthalen-5-ylamino)-1-methyl-cyclohexanol, Intermediate XLIV

A mixture of Intermediate **XXV-A** (200 mg, 0.84 mmol), cis-4-amino-1-methyl-cyclohexanol (130 mg, 1.01 mmol), sodium tert-butoxide (160 mg, 1.68 mmol), Pd₂(dba)₃ (50 mg, 0.084 mmol) and BINAP (0.06 eq, 30 mg, 0.050 mmol) in dioxane (8 mL) was heated in a sealed tube at 110 ºC for 3 h. The mixture was concentrated and the crude product was purified by column chromatography on silica gel using a solvent gradient from 0% to 6% of MeOH in DCM to give Intermediate **XLIV** (185 mg; 67%). LCMS (ESI): Rt = 0.37 min, m/z = 332.10 = [M+H]⁺.

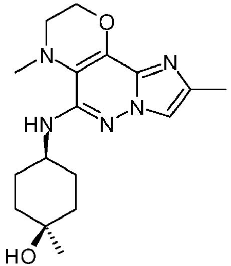

### 4-(3-Bromo-2,9-dimethyl-8,9-dihydro-7H-6-oxa-1,3a,4,9-tetraaza-cyclopenta[a]naphthalen-5-ylamino)-1-methyl-cyclohexanol, Intermediate XLV

To a solution of Intermediate **XLIV** (185 mg, 0.558 mmol) in chloroform (6 mL) was added NBS (100 mg, 0.564 mmol). The resulting mixture was stirred at rt for 30 min. The reaction mixture was diluted with DCM and washed several times with NaHCO₃ (sat sol), water and brine. The organic layer was dried over Na₂SO₄ and concentrated. The crude product was purified by column chromatography on silica gel using a solvent gradient from 0% to 2% of MeOH in DCM to give Intermediate 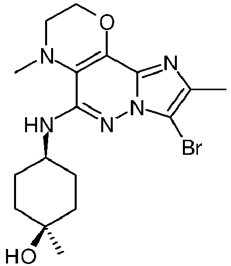 **XLV** (168 mg; 73%). LCMS (ESI): Rt = 3.35 min, m/z = 410.20/412.20 = [M+H]⁺.

### 4-[(2,7-Dimethyl-6,7-dihydro-8-oxa-1,3a,4-triaza-as-indacen-5-ylamino)-methyl]-4-fluoro-piperidine-1-carboxylic acid tert-butyl ester, Intermediate LII

Intermediate **XXI** (155 mg, 0.693 mmol), tert-butyl 4-(aminomethyl)-4-fluoropiperidine-I-carboxylate (241 mg, 1.040 mmol), BINAP (43 mg, 0.069 mmol), NaO^{t}Bu (133 mg, 1.386 mmol) and Pd₂(dba)₃ (63 mg, 0.069 mmol) were mixed in pressure tube and suspended in dioxane (7mL). The mixture was purged with Ar for few minutes and then heated at 110 ºC for 3 h 30 min. The mixture was taken in EtOAc and 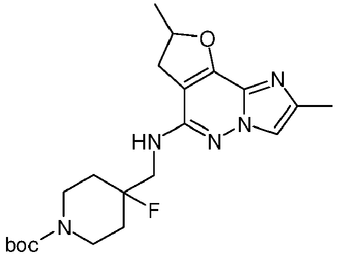 water. Layers were separated. The organic phase was dried and concentrated. The crude product was purified by column chromatography on silica gel using a solvent gradient from 20% to 100% of EtOAc in cHex affording Intermediate **LII** (foam, 258mg, 88%). LCMS (ESI): Rt = 3.35 min, m/z = 420.20 = [M+H]⁺. ¹H NMR (300 MHz, CDCl₃) δ ppm 7.41 (d, *J* = 1.0 Hz, 1H), 5.52 - 5.38 (m, 1H), 4.92 (m, 1H), 3.94 (m, 1H), 3.66 (dd, *J* = 20.7, 6.0 Hz, 2H), 3.41 (dd, *J* = 15.5, 9.7 Hz, 1H), 3.11 (t, *J* = 11.5 Hz, 2H), 2.83 (dd, *J* = 15.4, 7.4 Hz, 1H), 2.65 (d, *J* = 0.8 Hz, 3H), 1.88 (dd, *J* = 21.2, 11.0 Hz, 2H), 1.80 - 1.70 (m, 2H), 1.62 (d, *J* = 6.3 Hz, 3H), 1.46 (s, 9H).

### 4-[(3-Bromo-2,7-dimethyl-6,7-dihydro-8-oxa-1,3a,4-triaza-as-indacen-5-ylamino)-methyl]-4-fluoro-piperidine-1-carboxylic acid tert-butyl ester, Intermediate LIII

Intermediate **LII** (255 mg, 0.605 mmol) in chloroform (6 mL) was treated with NBS (119 mg, 0.666 mmol). The mixture was stirred at rt for 90 min. The reaction mixture was taken in DCM and washed with NaHCO₃ (sat sol). The organic phase was dried and concentrated. The crude product was purified by column chromatography on silica gel using a solvent gradient from 20% to 100% of EtOAc in cHex affording Intermediate **LIII** (solid, 90 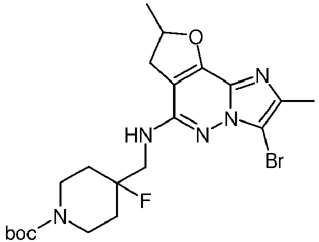 mg, 30%). LCMS (ESI): Rt = 4.47 min, m/z = 498.20/500.20 = [M+H]⁺. ¹H NMR (300 MHz, CDCl₃) δ ppm 5.35 - 5.20 (m, 1H), 4.44 (dd, *J* = 13.7, 7.6 Hz, 1H), 3.88 (d, *J* = 11.1 Hz, 2H), 3.70 (d, *J* = 20.4 Hz, 2H), 3.24 (dd, *J* = 14.5, 9.5 Hz, 1H), 3.13 (t, *J* = 11.1 Hz, 2H), 2.70 (dd, *J* = 14.5, 7.2 Hz, 1H), 2.37 (s, 3H), 1.94 - 1.73 (m, 3H), 1.72 - 1.58 (m, 1H), 1.53 (d, *J* = 6.3 Hz, 3H), 1.43 (s, 9H).

### 4-{[3-(2-Chloro-pyridin-4-yl)-2,7-dimethyl-6,7-dihydro-8-oxa-1,3a,4-triaza-as-indacen-5-ylamino]-methyl}-4-fluoro-piperidine-1-carboxylic acid tert-butyl ester, Intermediate LIV

Intermediate **LIII** (90 mg, 0.185 mmol), 2-chloropyridine-4-boronic acid (44 mg, 0.277 mmol), PdCl₂dppf (15 mg, 0.019 mmol) and Cs₂CO₃ (120 mg, 0.370 mmol) were mixed in dioxane (2 mL) and water (0.25 mL) in pressure tube. The mixture was heated at 110 ºC for 2 h. The mixture was taken in ethyl acetate and water. The aqueous layer was extracted with EtOAc. The combined organic extract was dried and 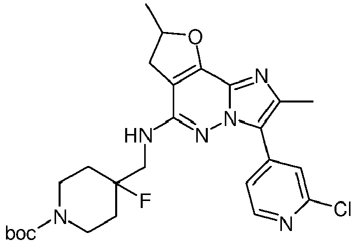 concentrated. The crude product was purified by column chromatography on silica gel using a solvent gradient from 20% to 100% of EtOAc in cHex and a second column using gradient from 40% to 100% of EtOAc in DCM to render Intermediate **LIV** (80 mg, 81%). LCMS (ESI): Rt = 3.32 min, m/z = 531.10 = [M+H]⁺.

### 5-Chloro-2,9-dimethyl-3-(2-trifluoromethyl-pyridin-4-yl)-8,9-dihydro-7H-6-oxa-1,3a,4,9-tetraaza-cyclopenta[a]naphthalene, Intermediate LV

A mixture of Intermediate **XLII** (75 mg, 0.23 mmol), 2-(trifluoromethyl)pyridine-4-boronic acid (55 mg,0.27 mmol), Pd(dppf)Cl₂ (15 mg, 0.018 mmol) and Cs₂CO₃ (491 mg, 1.508 mmol) in dioxane:H₂O (3 mL:1 mL) was heated at 120 ºC The dark mixture was cooled down, filtered through a Celite pad rinsing with DCM and concentrated under vacuum. The residue was purified by column 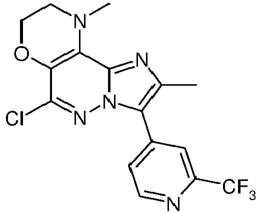 chromatography on silica gel using a solvent system from 15% to 75% of EtOAc in c-Hex, to give Intermediate **LV** (cream solid, 40 mg, 45%). LCMS (ESI): Rt =1.58 min, m/z = 384.00 [M+H]⁺. ¹H NMR (300 MHz, CDCl₃) δ ppm 8.74 (d, *J* = 5.2 Hz, 1H), 8.02 (d, *J* = 0.9 Hz, 1H), 7.87 (dd, *J* = 5.2, 1.4 Hz, 1H), 4.36 - 4.25 (m, 2H), 3.78 (s, 3H), 3.54 - 3.40 (m, 2H), 2.55 (s, 3H).

### B. Biological tests

**HASPIN biochemical Assay:** The biochemical assay to measure HASPIN activity relies on the ADP-Glo^{™} assay kit (Promega) that determines the amount of ADP as direct product of the kinase enzyme activity. Assay conditions were as indicated by the kit manufacturers with the following adaptations for the kinase activity step: Kinase assay buffer and assay volume (15 mM HEPES pH 7.5, 20 mM NaCl, 1 mM EGTA, 0.02% TWEEN 20, 10 mM MgCl₂, 0.1 mg/mL BGG)/25 µL assay volume). Incubation time and temperature: 60 min at 30 °C. HASPIN final concentration: 0.9 µg/mL. ATP final concentration: 150 µM. HASPIN autophosphorylation was measured. Assays were performed in 384-well plates. The final outcome of the coupled reactions provided by the kit is the release of luciferase and has been measured with a multilabel HTS counter Victor V / Envision. Values were normalized against the control activity included (100% HASPIN activity, without compound). Values were plotted against the inhibitor concentration and fit to a sigmoid dose-response curve using Activity base by IDBS software.

**Cellular HASPIN Inhibition Assay (phosphorylation of H3T3):** Compounds can be screened for their ability to inhibit intracellular HASPIN using a western blot assay to detect phosphorylation of the HASPIN substrate H3T3 in synchronized cells. MV4:11 cells are plated at 400000 cells per well in 6-well plates in RPMI media (Sigma-Aldrich R6504) supplemented with 10% foetal bovine serum (Sigma-Aldrich F7524), Penicillin/Streptomycin solution diluted 1: 100 (Gibco 15070-063), and fungizone (Gibco, 15290-018), and allowed to adhere overnight at 37 ºC in 5% CO₂. Then, compounds are added to the cell media from a final concentration of 10 µM in 10-fold serial dilutions and the cells are incubated at 37 ºC in 5% CO₂. After 8 hours of treatment with the compounds, the cells are washed in PBS, lysed adding 100 µl of protein lysis buffer (62.5 mM Tris pH 6.8 al 6.25%, 2% SDS y 10% glycerol) incubation 10 minutes at room temperature and heating at 95 ºC 10 min. The protein content of the lysates is determined by DC protein assay (Biorad, Ref. 5000116). The proteins are resolved by SDS-PAGE and transferred to nitrocellulose membrane (VWR International Eurolab, Ref. 732-4007). The membranes are incubated overnight at 4 ºC with antibodies specific for H3 (Millipore #07424), phosphothreonine-3 H3 (Cell Signaling Ref.14269) they are washed and then incubated with IRDye800 conjugated anti-mouse (Pierce/Cultek, 35521) and Alexa Fluor 680 goat anti-rabbit IgG secondary antibodies (Invitrogen, A21076). The bands are visualized and quantified using an Odyssey infrared imaging system. The percentage of H3 phosphorylation is finally plotted against concentration for each compound and EC₅₀S for intracellular HASPIN inhibition are calculated using ActivityBase from IDBS.

Compounds of the invention were found to inhibit HASPIN, as tested in the biochemical assay described hereinbefore, with IC₅₀ activities below 5 µM. Biological activity in HASPIN is represented in Table 1.

**Table 1: Inhibition of HASPIN activity expressed as IC₅₀ (biochemical) and EC₅₀ (cellular) values [M] for some compounds of the examples.**

| **Cpd number** | **HASPIN IC₅₀** | **pH3T3 EC₅₀** | **Cpd number** | **HASPIN IC₅₀** | **pH3T3 EC₅₀** |
|---|---|---|---|---|---|
| 1 | 4.80E-09 | < 5.0E-08 | 17 | 2.25E-07 | |
| 2 | 5.03E-09 | < 5.0E-08 | 18 | 2.31 E-07 | |
| 3 | 1.02E-08 | < 5.0E-08 | 25 | 2.23E-08 | < 5.0E-08 |
| 4 | 2.47E-08 | < 5.0E-08 | 27 | 7.52E-08 | |
| 5 | 3.65E-08 | | 28 | 1.01 E-07 | |
| 6 | 5.32E-08 | | 31 | 2.21 E-07 | |
| 7 | 4.02E-08 | | 33 | 6.37E-09 | |
| 10 | 5.05E-08 | | 34 | 5.87E-09 | < 5.0E-08 |
| 12 | 6.13E-08 | | 35 | 2.07E-08 | < 5.0E-08 |
| 13 | 6.98E-08 | | 33 | 6.37E-09 | |
| 14 | 1.33E-07 | | 34 | 5.87E-09 | < 5.0E-08 |
| 15 | 1.45E-07 | | 35 | 2.07E-08 | < 5.0E-08 |

## Claims

1. A compound of formula (I): 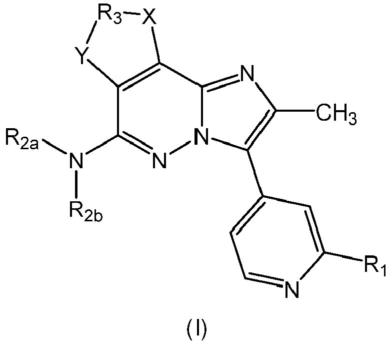 wherein:
R₁ is selected from the following groups: H, halo, CF₃,
R₂ₐ and R_{2b} are independently selected from the following groups:
- H
- alkyl C₁-C₆ substituted by OH or by heterocycle, said heterocycle being optionally substituted by halo;
- cycloalkyl C₃-C₆ substituted by OH or by alkyl C₁-C₄;
or R₂ₐ and R_{2b} are linked and form a cycle together with the N atom to which they are attached, and R₂ₐ and R_{2b} are the same or different alkylene C₁-C₃ optionally substituted by an alkyl C₁-C₄, said alkyl being optionally substituted by OH or by NH₂;
X and Y are independently selected from O, CH₂, N(alkyl C₁-C₄) and R₃ is an alkylene C₁-C₂ optionally substituted by alkyl C₁-C₄, with the proviso that one of X and Y must be O and that the cycle formed by X, Y and R₃ is not aromatic;
or a pharmaceutically acceptable ester, amide, solvate or salt thereof.

2. The compound according to any claim 1, wherein R₁ is selected from Cl, F, CF₃.

3. The compound according to any one of claims 1 or 2, wherein R₂ₐ is H and R_{2b} is selected from alkyl C₁-C₄ substituted by OH or 4-piperidinyl optionally substituted by F.

4. The compound according to any one of claims 1 or 2, wherein R₂ₐ is H and R_{2b} is cycloalkyl C₆ substituted by OH and by alkyl C₁-C₂;

5. The compound according to any one of claims 1 or 2, wherein R₂ₐ and R_{2b} are linked and form a cycle together with the N atom to which they are attached, and R₂ₐ is alkylene C₃ and R_{2b} is alkylene C₂ optionally substituted by alkyl C₁-C₂ optionally substituted by NH₂.

6. The compound according to any one of claims 1 to 5 wherein X and Y are independently selected from O, CH₂, N-CH₃ and R₃ is an alkylene C₂ or alkylene C₁ optionally substituted by methyl.

7. The compound according to claim 1, wherein said compound is selected from the following list:
- [3-(2-Chloro-pyridin-4-yl)-2-methyl-7,8-dihydro-6H-9-oxa-1,3a,4-triaza-cyclopenta[a]naphthalen-5-yl]-(4-fluoro-piperidin-4-ylmethyl)-amine (1)
- C-{1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (2)
- C-{(S)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (3)
- C-{(R)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (4)
- 4-[3-(2-Chloro-pyridin-4-yl)-2-methyl-7,8-dihydro-6H-9-oxa-1,3a,4-triaza-cyclopenta[a]naphthalen-5-ylamino]-1-methyl-cyclohexanol (5)
- (4-Fluoro-piperidin-4-ylmethyl)-(2-methyl-3-pyridin-4-yl-7,8-dihydro-6H-9-oxa-1,3a,4-triaza-cyclopenta[a]naphthalen-5-yl)-amine (6)
- 4-[3-(2-Chloro-pyridin-4-yl)-2,7-dimethyl-6,7-dihydro-8-oxa-1,3a,4-triaza-as-indacen-5-ylamino]-1-methyl-cyclohexanol (7)
- [3-(2-Chloro-pyridin-4-yl)-2,6-dimethyl-7,8-dihydro-6H-9-oxa-1,3a,4,6-tetraaza-cyclopenta[a]naphthalen-5-yl]-piperidin-4-ylmethyl-amine (10)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-butan-1-ol (12)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (13)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (14)
- [2,6-Dimethyl-3-(2-trifluoromethyl-pyridin-4-yl)-7,8-dihydro-6H-9-oxa-1,3a,4,6-tetraaza-cyclopenta[a]naphthalen-5-yl]-piperidin-4-ylmethyl-amine (15)
- (2,6-Dimethyl-3-pyridin-4-yl-7,8-dihydro-6H-9-oxa-1,3a,4,6-tetraaza-cyclopenta[a]naphthalen-5-yl)-piperidin-4-ylmethyl-amine (17)
- 4-[3-(2-Fluoro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (18)
- [3-(2-Chloro-pyridin-4-yl)-2,9-dimethyl-8,9-dihydro-7H-6-oxa-1,3a,4,9-tetraaza-cyclopenta[a]naphthalen-5-yl]-(4-fluoro-piperidin-4-ylmethyl)-amine (25)
- 4-(2,9-Dimethyl-3-pyridin-4-yl-8,9-dihydro-7H-6-oxa-1,3a,4,9-tetraaza-cyclopenta[a]naphthalen-5-ylamino)-1-methyl-cyclohexanol (27)
- 4-[3-(2-Chloro-pyridin-4-yl)-2,9-dimethyl-8,9-dihydro-7H-6-oxa-1,3a,4,9-tetraaza-cyclopenta[a]naphthalen-5-ylamino]-1-methyl-cyclohexanol (28)
- 4-[2,9-Dimethyl-3-(2-trifluoromethyl-pyridin-4-yl)-8,9-dihydro-7H-6-oxa-1,3a,4,9-tetraaza-cyclopenta[a]naphthalen-5-ylamino]-1-methyl-cyclohexanol (29)
- [3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (33)
- [(R)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (34)
- [(S)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (35).

8. The compound according to claim 1, wherein said compound is selected from the following list:
- [3-(2-Chloro-pyridin-4-yl)-2-methyl-7,8-dihydro-6H-9-oxa-1,3a,4-triaza-cyclopenta[a]naphthalen-5-yl]-(4-fluoro-piperidin-4-ylmethyl)-amine (1)
- C-{1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (2)
- C-{(S)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (3)
- C-{(R)-1-[3-(2-Chloro-pyridin-4-yl)-2-methyl-7,8-dihydro-6H-9-oxa-1,3a,4-triaza-cyclopenta[a]naphthalen-5-yl]-piperidin-3-yl}-methylamine (4)
- 4-[3-(2-Chloro-pyridin-4-yl)-2-methyl-7,8-dihydro-6H-9-oxa-1,3a,4-triaza-cyclopenta[a]naphthalen-5-ylamino]-1-methyl-cyclohexanol (5)
- (4-Fluoro-piperidin-4-ylmethyl)-(2-methyl-3-pyridin-4-yl-7,8-dihydro-6H-9-oxa-1,3a,4-triaza-cyclopenta[a]naphthalen-5-yl)-amine (6)
- 4-[3-(2-Chloro-pyridin-4-yl)-2,7-dimethyl-6,7-dihydro-8-oxa-1,3a,4-triaza-as-indacen-5-ylamino]-1-methyl-cyclohexanol (7)
- [3-(2-Chloro-pyridin-4-yl)-2,6-dimethyl-7,8-dihydro-6H-9-oxa-1,3a,4,6-tetraaza-cyclopenta[a]naphthalen-5-yl]-piperidin-4-ylmethyl-amine (10)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-butan-1-ol (12)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (13)
- 4-[3-(2-Chloro-pyridin-4-yl)-2,6-dimethyl-7,8-dihydro-6H-9-oxa-1,3a,4,6-tetraaza-cyclopenta[a]naphthalen-5-ylamino]-1-methyl-cyclohexanol (14)
- [3-(2-Chloro-pyridin-4-yl)-2,9-dimethyl-8,9-dihydro-7H-6-oxa-1,3a,4,9-tetraaza-cyclopenta[a]naphthalen-5-yl]-(4-fluoro-piperidin-4-ylmethyl)-amine (25)
- 4-(2,9-Dimethyl-3-pyridin-4-yl-8,9-dihydro-7H-6-oxa-1,3a,4,9-tetraaza-cyclopenta[a]naphthalen-5-ylamino)-1-methyl-cyclohexanol (27)
- 4-[3-(2-Chloro-pyridin-4-yl)-2,9-dimethyl-8,9-dihydro-7H-6-oxa-1,3a,4,9-tetraaza-cyclopenta[a]naphthalen-5-ylamino]-1-methyl-cyclohexanol (28)
- [3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (33)
- [(R)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (34)
- [(S)-3-(2-Chloro-pyridin-4-yl)-2,7-dimethyl-6,7-dihydro-8-oxa-1,3a,4-triaza-as-indacen-5-yl]-(4-fluoro-piperidin-4-ylmethyl)-amine (35).

9. A compound of formula (I) according to any one of claims 1 to 8 for use as a medicament.

10. A compound of formula (I) according to any one of claims 1 to 8 for use in the prevention or treatment of cancer.

11. The compound for use according to claim 10, wherein the cancer is selected from Burkitt's lymphoma, chronic lymphocytic leukemias, pancreatic cancer, gallbladder carcinoma, bladder cancer, prostate cancer, melanoma, breast cancer, ovarian cancer.

12. A composition comprising a compound of formula (I) according to any one of claims 1 to 8 and a pharmaceutically acceptable excipient, diluent or carrier.
